# EUROPEAN PATENT SPECIFICATION

(11) **EP 2 066 799 B1**
(45) Date of publication and mention of the grant of the patent: **11.12.2013**
(21) Application number: 07829258.8
(22) Date of filing: 28.09.2007
(51) Int. Cl.: C12P 13/06, C12N 9/02

(54) **METHOD FOR PRODUCING 4-HYDROXY-L-ISOLEUCINE**
VERFAHREN ZUR HERSTELLUNG VON 4-HYDROXY-L-ISOLEUCIN
MÉTHODE DE PRODUCTION DE 4-HYDROXY-L-ISOLEUCINE

(30) Priority: 28.09.2006 JP 2006265452; 16.10.2006 US 829577 P; 22.12.2006 JP 2006345461; 07.02.2007 RU 2007104645
(43) Date of publication of application: 10.06.2009
(73) Proprietor: Ajinomoto Co., Inc., Tokyo 104-8315 (JP)
(72) Inventor: KODERA, Tomohiro, Kawasaki-shi, Kanagawa 2108681 (JP); SMIRNOV, Sergey Vasilievich, Moscow 121552 (RU); SAMSONOVA, Natalia Nikolaevna, Moscow 117186 (RU); KOTLIAROVA, Veronika Aleksandrovna, Moscow 117574 (RU); RUSHKEVICH, Natalia Yurievna, Moscow 121170 (RU); KOZLOV, Yury Ivanovich, Moscow 117574 (RU); SHIMIZU, Sakayu, Kyoto-shi, Kyoto 6068502 (JP); OGAWA, Jun, Kyoto-shi, Kyoto 6068502 (JP); HIBI, Makoto, Kyoto-shi, Kyoto 6068502 (JP)
(74) Representative: Strehl Schübel-Hopf & Partner
(86) International application number: PCT/JP2007/069520
(87) International publication number: WO 2008/044614

(56) References cited:
- WO-A-2006/093322
- HAEFELE C ET AL: "CHARACTERIZATION OF A DIOXYGENASE FROM TRIGONELLA FOENUM-GRAECUM INVOLVED IN 4-HYDROXYISOLEUCINE BIOSYNTHESIS" PHYTOCHEMISTRY, PERGAMON PRESS, GB, vol. 44, no. 4, 1 February 1997 (1997-02-01), pages 563-566, XP000675411 ISSN: 0031-9422 cited in the application
- DATABASE UNIPROTKB 8 November 2005 (2005-11-08), ANDERSON, I. ET AL.: "Comparative genome analysis of Bacillus cereus group genomes with Bacillus subtilis" XP002469777 Database accession no. Q3EZS9
- DATABASE UNIPROTKB 1 June 2003 (2003-06-01), IVANOVA,N. ET AL.: "Genome sequence of Bacillus cereus and comparative analysis with Bacillus anthracis" XP002469778 Database accession no. Q81GX0
- DATABASE EMBL 1 January 2006 (2006-01-01), IVANOVA,N. ET AL.: "Genome sequence of Bacillus cereus and comparative analysis with Bacillus anthracis" XP002469779 Database accession no. AE016877
- DE CAROLIS EMIDIO ET AL: "2-Oxoglutarate-dependent dioxygenase and related enzymes: Biochemical characterization" PHYTOCHEMISTRY (OXFORD), vol. 36, no. 5, 1994, pages 1093-1107, XP002469775 ISSN: 0031-9422
- NARENDER ET AL: "4-Hydroxyisoleucine an unusual amino acid as antidyslipidemic and antihyperglycemic agent" BIOORGANIC & MEDICINAL CHEMISTRY LETTERS, OXFORD, GB, vol. 16, no. 2, 15 January 2006 (2006-01-15), pages 293-296, XP005174984 ISSN: 0960-894X
- SMIRNOV S V ET AL: "A novel strategy for enzymatic synthesis of 4-hydroxyisoleucine: identification of an enzyme possessing HMKP (4-hydroxy-3-methyl-2-ket o-pentanoate) aldolase activity" FEMS MICROBIOLOGY LETTERS, AMSTERDAM, NL, vol. 273, no. 1, August 2007 (2007-08), pages 70-77, XP002442257 ISSN: 0378-1097
- OGAWA JUN ET AL: "Synthesis of 4-hydroxyisoleucine by the aldolase-transaminase coupling reaction and basic characterization of the aldolase from Arthrobacter simplex AKU 626" BIOSCIENCE BIOTECHNOLOGY AND BIOCHEMISTRY, vol. 71, no. 7, July 2007 (2007-07), pages 1607-1615, XP002469776 ISSN: 0916-8451

### SEQUENCE LISTING

<110> Ajinomoto Co., Inc.
<120> METHOD FOR PRODUCING 4-HYDROXY-L-ISOLEUCINE
<130> EPA-64694
<150> JP 2006-265452
   <151> 2006-09-28
<150> JP 2006-345461
   <151> 2006-12-22
<150> RU 2007104645
   <151> 2007-02-07
<150> US 60/829577
   <151> 2006-10-16
<160> 21
<170> PatentIn version 3.1
<210> 1
   <211> 717
   <212> DNA
   <213> Bacillus thuringiensis strain 2-e-2
<220>
   <221> CDS
   <222> (1)..(717)
   <223>
<400> 1
   aaa atg agt ggc ttt agc ata gaa gaa aag gta cat gaa ttt gaa tct     48
   Lys Met Ser Gly Phe Ser Ile Glu Glu Lys Val His Glu Phe Glu Ser
   1               5                   10                  15
   aaa ggg ttt ctt gaa atc tca aat gaa atc ttt tta caa gag gaa gag     96
   Lys Gly Phe Leu Glu Ile Ser Asn Glu Ile Phe Leu Gln Glu Glu Glu
               20                  25                  30
   aat cat agt tta tta aca caa gca cag tta gat tat tat aat ttg gaa     144
   Asn His Ser Leu Leu Thr Gln Ala Gln Leu Asp Tyr Tyr Asn Leu Glu
           35                  40                  45
   gat gat gcg tac ggt gaa tgc cgt gct aga tct tat tca agg tat ata     192
   Asp Asp Ala Tyr Gly Glu Cys Arg Ala Arg Ser Tyr Ser Arg Tyr Ile
       50                  55                  60
   aag tat gtt gat tca cca gat tat att tta gat aat agt aat gat tac     240
   Lys Tyr Val Asp Ser Pro Asp Tyr Ile Leu Asp Asn Ser Asn Asp Tyr
   65                  70                  75                  80
   ttc caa tct aaa gaa tat aac tat gat gat ggc ggg aaa gtt aga cag     288
   Phe Gln Ser Lys Glu Tyr Asn Tyr Asp Asp Gly Gly Lys Val Arg Gln
                   85                  90                  95
   ttc aat agc ata aat gat agc ttt tta tgt aat cct tta att caa aat     336
   Phe Asn Ser Ile Asn Asp Ser Phe Leu Cys Asn Pro Leu Ile Gln Asn
               100                 105                 110
   atc gtg cgt ttc gat act gag ttt gca ttt aaa aca aat ata ata gat     384
   Ile Val Arg Phe Asp Thr Glu Phe Ala Phe Lys Thr Asn Ile Ile Asp
           115                 120                 125
   aaa agt aaa gat tta att ata ggc tta cat caa gta aga tat aaa gct     432
   Lys Ser Lys Asp Leu Ile Ile Gly Leu His Gln Val Arg Tyr Lys Ala
       130                 135                 140
   act aaa gaa aga cca tct ttt agt tca cct att tgg tta cat aaa gat     480
   Thr Lys Glu Arg Pro Ser Phe Ser Ser Pro Ile Trp Leu His Lys Asp
   145                 150                 155                 160
   gat gaa cca gta gta ttt tta cac ctt atg aat tta agt aat aca gct     528
   Asp Glu Pro Val Val Phe Leu His Leu Met Asn Leu Ser Asn Thr Ala
                   165                 170                 175
   atc ggc gga gat aat tta ata gct aat tct cct cgg gaa att aat cag     576
   Ile Gly Gly Asp Asn Leu Ile Ala Asn Ser Pro Arg Glu Ile Asn Gln
               180                 185                 190
   ttt ata agt ttg aag gag cct tta gaa act tta gta ttt gga caa aag     624
   Phe Ile Ser Leu Lys Glu Pro Leu Glu Thr Leu Val Phe Gly Gln Lys
           195                 200                 205
   gtc ttc cat gcc gta acg cca ctt gga aca gaa tgt agt acg gag gct     672
   Val Phe His Ala Val Thr Pro Leu Gly Thr Glu Cys Ser Thr Glu Ala
       210                 215                 220
   ttt cgt gat att tta tta gta aca ttt tct tat aag gag aca aaa         717
   Phe Arg Asp Ile Leu Leu Val Thr Phe Ser Tyr Lys Glu Thr Lys
   225                 230                 235
<210> 2
   <211> 239
   <212> PRT
   <213> Bacillus thuringiensis FERM BP-10688
<400> 2
   Lys Met Ser Gly Phe Ser Ile Glu Glu Lys Val His Glu Phe Glu Ser
   1               5                   10                  15
   Lys Gly Phe Leu Glu Ile Ser Asn Glu Ile Phe Leu Gln Glu Glu Glu
               20                  25                  30
   Asn His Ser Leu Leu Thr Gln Ala Gln Leu Asp Tyr Tyr Asn Leu Glu
           35                  40                  45
   Asp Asp Ala Tyr Gly Glu Cys Arg Ala Arg Ser Tyr Ser Arg Tyr Ile
       50                  55                  60
   Lys Tyr Val Asp Ser Pro Asp Tyr Ile Leu Asp Asn Ser Asn Asp Tyr
   65                  70                  75                  80
   Phe Gln Ser Lys Glu Tyr Asn Tyr Asp Asp Gly Gly Lys Val Arg Gln
                   85                  90                  95
   Phe Asn Ser Ile Asn Asp Ser Phe Leu Cys Asn Pro Leu Ile Gln Asn
               100                 105                 110
   Ile Val Arg Phe Asp Thr Glu Phe Ala Phe Lys Thr Asn Ile Ile Asp
           115                 120                 125
   Lys Ser Lys Asp Leu Ile Ile Gly Leu His Gln Val Arg Tyr Lys Ala
       130                 135                 140
   Thr Lys Glu Arg Pro Ser Phe Ser Ser Pro Ile Trp Leu His Lys Asp
   145                 150                 155                 160
   Asp Glu Pro Val Val Phe Leu His Leu Met Asn Leu Ser Asn Thr Ala
                   165                 170                 175
   Ile Gly Gly Asp Asn Leu Ile Ala Asn Ser Pro Arg Glu Ile Asn Gln
               180                 185                 190
   Phe Ile Ser Leu Lys Glu Pro Leu Glu Thr Leu Val Phe Gly Gln Lys
           195                 200                 205
   Val Phe His Ala Val Thr Pro Leu Gly Thr Glu Cys Ser Thr Glu Ala
       210                 215                 220
   Phe Arg Asp Ile Leu Leu Val Thr Phe Ser Tyr Lys Glu Thr Lys
   225                 230                 235
<210> 3
   <211> 65
   <212> DNA
   <213> Artificial sequence
<220>
   <223> primer SVS 170
<400> 3
   ctctagagga tccttaagaa ggagatatac catgaaaatg agtggcttta gcatagaaga    60
   aaagg                                                                65
<210> 4
   <211> 35
   <212> DNA
   <213> Artificial sequence
<220>
   <223> primer SVS 169
<400> 4
   gaattcgagc tcttattttg tctccttata agaaa 35
<210> 5
   <211> 20
   <212> PRT
   <213> Bacillus thuringiensis FERM BP-10688
<400> 5
   Lys Met Ser Gly Phe Ser Ile Glu Glu Lys Val His Glu Phe Glu Ser
   1               5                   10                  15
   Lys Gly Phe Leu
               20
<210> 6
   <211> 239
   <212> PRT
   <213> Artificial sequence
<220>
   <223> conserved sequence
<220>
   <221> MISC_FEATURE
   <222> (1)..(239)
   <223> Xaa = any amino acid
<400> 6
   Lys Xaa Xaa Xaa Phe Xaa Xaa Glu Xaa Lys Val His Glu Phe Glu Ser
   1               5                   10                  15
   Xaa Gly Xaa Xaa Xaa Ile Xaa Asn Xaa Ile Phe Leu Gln Xaa Xaa Glu
               20                  25                  30
   Xaa Xaa Xaa Leu Leu Thr Xaa Ala Gln Leu Asp Tyr Tyr Xaa Leu Xaa
           35                  40                  45
   Xaa Asp Ala Tyr Gly Glu Cys Arg Ala Arg Xaa Tyr Ser Arg Tyr Ile
       50                  55                  60
   Lys Tyr Xaa Xaa Ser Xaa Asp Tyr Xaa Leu Asp Xaa Xaa Asn Xaa Tyr
   65                  70                  75                  80
   Phe Gln Ser Xaa Glu Tyr Asn Tyr Asp Asp Gly Gly Lys Xaa Arg Xaa
                   85                  90                  95
   Phe Xaa Ser Ile Xaa Asp Xaa Phe Leu Xaa Asn Xaa Leu Ile Xaa Xaa
               100                 105                 110
   Ile Val Arg Phe Asp Xaa Glu Phe Ala Xaa Xaa Thr Asn Ile Xaa Asp
           115                 120                 125
   Thr Ser Lys Asp Leu Xaa Ile Gly Leu His Gln Val Arg Tyr Lys Ala
       130                 135                 140
   Thr Xaa Glu Xaa Pro Ser Phe Ser Ser Pro Ile Trp Leu His Lys Asp
   145                 150                 155                 160
   Asp Glu Pro Xaa Val Phe Leu His Leu Met Asn Leu Ser Asn Thr Ala
                   165                 170                 175
   Xaa Gly Gly Asp Asn Leu Ile Ala Asn Ser Pro Arg Glu Ile Asn Xaa
               180                 185                 190
   Xaa Ile Ser Leu Lys Xaa Pro Leu Glu Thr Leu Val Phe Gly Gln Lys
           195                 200                 205
   Val Phe His Ala Val Thr Pro Leu Gly Thr Glu Cys Xaa Thr Glu Ala
       210                 215                 220
   Xaa Arg Asp Ile Leu Leu Val Thr Phe Ser Tyr Lys Glu Xaa Lys
   225                 230                 235
<210> 7
   <211> 720
   <212> DNA
   <213> Bacillus thuringiensis (serovar israelensis); ATCC 35646
<220>
   <221> CDS
   <222> (1)..(720)
   <223>
<400> 7
   aaa atg agt ggc ttt agc ata gaa gaa aag gta cat gaa ttt gaa tct        48
   Lys Met Ser Gly Phe Ser Ile Glu Glu Lys Val His Glu Phe Glu Ser
   1               5                   10                  15
   aaa ggg ttt ctt gaa atc tca aat gaa atc ttt tta caa gag gaa gag        96
   Lys Gly Phe Leu Glu Ile Ser Asn Glu Ile Phe Leu Gln Glu Glu Glu
               20                  25                  30
   aat cat agt tta tta aca caa gca cag tta gat tat tat aat ttg gaa       144
   Asn His Ser Leu Leu Thr Gln Ala Gln Leu Asp Tyr Tyr Asn Leu Glu
           35                  40                  45
   gat gat gcg tac ggt gaa tgc cgt gct aga tct tat tca agg tat ata       192
   Asp Asp Ala Tyr Gly Glu Cys Arg Ala Arg Ser Tyr Ser Arg Tyr Ile
       50                  55                  60
   aag tat gtt gat tca cca gat tat att tta gat aat agt aat gat tac       240
   Lys Tyr Val Asp Ser Pro Asp Tyr Ile Leu Asp Asn Ser Asn Asp Tyr
   65                  70                  75                  80
   ttc caa tct aaa gaa tat aac tat gat gat ggc ggg aaa gtt aga cag       288
   Phe Gln Ser Lys Glu Tyr Asn Tyr Asp Asp Gly Gly Lys Val Arg Gln
                   85                  90                  95
   ttc aat agc ata aat gat agc ttt ttg tgt aat cct tta att caa aat       336
   Phe Asn Ser Ile Asn Asp Ser Phe Leu Cys Asn Pro Leu Ile Gln Asn
               100                 105                 110
   atc gtg cat ttc gat act gag ttt gca ttt aaa aca aat ata ata gat       384
   Ile Val His Phe Asp Thr Glu Phe Ala Phe Lys Thr Asn Ile Ile Asp
           115                 120                 125
   aaa agt aaa gat tta att ata ggc tta cat caa gta aga tat aaa gct       432
   Lys Ser Lys Asp Leu Ile Ile Gly Leu His Gln Val Arg Tyr Lys Ala
       130                 135                 140
   act aaa gaa aga cca tct ttt agt tca cct att tgg tta cat aaa gat       480
   Thr Lys Glu Arg Pro Ser Phe Ser Ser Pro Ile Trp Leu His Lys Asp
   145                 150                 155                 160
   gat gaa cca gta gtg ttt tta cac ctt atg aat tta agt aat aca gct       528
   Asp Glu Pro Val Val Phe Leu His Leu Met Asn Leu Ser Asn Thr Ala
                   165                 170                 175
   atc ggc gga gat aat tta ata gct aat tct cct cgg gaa att aat cag       576
   Ile Gly Gly Asp Asn Leu Ile Ala Asn Ser Pro Arg Glu Ile Asn Gln
               180                 185                 190
   ttt ata agt ttg aag gag ccg tta gaa act tta gta ttt gga caa aag       624
   Phe Ile Ser Leu Lys Glu Pro Leu Glu Thr Leu Val Phe Gly Gln Lys
           195                 200                 205
   gtc ttc cat gcc gta acg cca ctt gga aca gaa tgt agt acg gag gct       672
   Val Phe His Ala Val Thr Pro Leu Gly Thr Glu Cys Ser Thr Glu Ala
       210                 215                 220
   ttt cgt gat att tta tta gta aca ttt tct tat aag gag aca aaa taa       720
   Phe Arg Asp Ile Leu Leu Val Thr Phe Ser Tyr Lys Glu Thr Lys
   225                 230                 235
<210> 8
   <211> 239
   <212> PRT
   <213> Bacillus thuringiensis (serovar israelensis); ATCC 35646
<400> 8
   Lys Met Ser Gly Phe Ser Ile Glu Glu Lys Val His Glu Phe Glu Ser
   1               5                   10                  15
   Lys Gly Phe Leu Glu Ile Ser Asn Glu Ile Phe Leu Gln Glu Glu Glu
               20                  25                  30
   Asn His Ser Leu Leu Thr Gln Ala Gln Leu Asp Tyr Tyr Asn Leu Glu
           35                  40                  45
   Asp Asp Ala Tyr Gly Glu Cys Arg Ala Arg Ser Tyr Ser Arg Tyr Ile
       50                  55                  60
   Lys Tyr Val Asp Ser Pro Asp Tyr Ile Leu Asp Asn Ser Asn Asp Tyr
   65                  70                  75                  80
   Phe Gln Ser Lys Glu Tyr Asn Tyr Asp Asp Gly Gly Lys Val Arg Gln
                   85                  90                  95
   Phe Asn Ser Ile Asn Asp Ser Phe Leu Cys Asn Pro Leu Ile Gln Asn
               100                 105                 110
   Ile Val His Phe Asp Thr Glu Phe Ala Phe Lys Thr Asn Ile Ile Asp
           115                 120                 125
   Lys Ser Lys Asp Leu Ile Ile Gly Leu His Gln Val Arg Tyr Lys Ala
       130                 135                 140
   Thr Lys Glu Arg Pro Ser Phe Ser Ser Pro Ile Trp Leu His Lys Asp
   145                 150                 155                 160
   Asp Glu Pro Val Val Phe Leu His Leu Met Asn Leu Ser Asn Thr Ala
                   165                 170                 175
   Ile Gly Gly Asp Asn Leu Ile Ala Asn Ser Pro Arg Glu Ile Asn Gln
               180                 185                 190
   Phe Ile Ser Leu Lys Glu Pro Leu Glu Thr Leu Val Phe Gly Gln Lys
           195                 200                 205
   Val Phe His Ala Val Thr Pro Leu Gly Thr Glu Cys Ser Thr Glu Ala
       210                 215                 220
   Phe Arg Asp Ile Leu Leu Val Thr Phe Ser Tyr Lys Glu Thr Lys
   225                 230                 235
<210> 9
   <211> 537
   <212> DNA
   <213> Bacillus thuringiensis
<400> 9
   tggagagttt gatcctggct caggatgaac gctggcggcg tgcctaatac atgcaagtcg    60
   agcgaatgga ttgagagctt gctctcaaga agttagcggc ggacgggtga gtaacacgtg   120
   ggtaacctgc ccataagact gggataactc cgggaaaccg gggctaatac cggataacat   180
   tttgaactgc atggttcgaa attgaaaggc ggcttcggct gtcacttatg gatggacccg   240
   cgtcgcatta gctagttggt gaggtaacgg ctcaccaagg caacgatgcg tagccgacct   300
   gagagggtga tcggccacac tgggactgag acacggccca gactcctacg ggaggcagca   360
   gtagggaatc ttccgcaatg gacgaaagtc tgacggagca acgccgcgtg agtgatgaag   420
   gctttcgggt cgtaaaactc tgttgttagg gaagaacaag tgctagttga ataagctggc   480
   accttgacgg tacctaacca gaaagccacg gctaactacg tgccagcagc cgcggta      537
<210> 10
   <211> 30
   <212> DNA
   <213> Artificial Sequence
<220>
   <223> Primer
<400> 10
   catatggagg tttttataat gacgtttgtt 30
<210> 11
   <211> 30
   <212> DNA
   <213> Artificial Sequence
<220>
   <223> Primer
<400> 11
   ctcgagtttt gtctccttat aagaaaatgt 30
<210> 12
   <211> 753
   <212> DNA
   <213> Bacillus cereus ATCC14579
<400> 12
   gtggaggttt ttataatgac gtttgttctt agtaaaatga atgggtttag catagaagaa     60
   aaggtacatg aatttgaatc taaaggattc cttgaaatct caaatgaaat ctttttacaa    120
   gaggaagaga atcatcgttt attaacacaa gcacagttag attattataa tttggaagat    180
   gatgcgtacg gtgaatgccg tgctagatct tattcaaggt atataaagta tgttgattca    240
   ccagattata ttttagataa tagtaatgat tacttccaat ctaaagaata taactatgac    300
   gatggcggga aagttagaca gttcaatagc ataaatgata gctttttatg caatccttta    360
   attcaaaata tcgtgcgttt cgatactgaa tttgcattta aaacaaatat aatagataca    420
   agtaaagact taattatagg tttacatcaa gtaagatata aagctactaa agaaagacca    480
   tcttttagtt cacctatttg gttacataaa gatgatgaac cagtagtatt tttacacctt    540
   atgaatttaa gtaatacagc tattggtgga gataatttaa tagctaattc tcctcgagaa    600
   attaatcagt ttataagttt gaaggagcct ttagaaactt tagtatttgg acaaaaggtc    660
   ttccatgccg taacgccact tggaacagaa tgtagtacgg aggcttttcg tgatatttta    720
   ttagtaacat tttcttataa ggagacaaaa tga                                 753
<210> 13
   <211> 250
   <212> PRT
   <213> Bacillus cereus ATCC14579
<400> 13
   Met Glu Val Phe Ile Met Thr Phe Val Leu Ser Lys Met Asn Gly Phe
   1               5                   10                  15
   Ser Ile Glu Glu Lys Val His Glu Phe Glu Ser Lys Gly Phe Leu Glu
                   20                      25                      30
   Ile Ser Asn Glu Ile Phe Leu Gln Glu Glu Glu Asn His Arg Leu Leu
               35                      40                      45
   Thr Gln Ala Gln Leu Asp Tyr Tyr Asn Leu Glu Asp Asp Ala Tyr Gly
       50                      55                      60
   Glu Cys Arg Ala Arg Ser Tyr Ser Arg Tyr Ile Lys Tyr Val Asp Ser
   65                      70                      75                      80
   Pro Asp Tyr Ile Leu Asp Asn Ser Asn Asp Tyr Phe Gln Ser Lys Glu
                   85                      90                      95
   Tyr Asn Tyr Asp Asp Gly Gly Lys Val Arg Gln Phe Asn Ser Ile Asn
               100                     105                     110
   Asp Ser Phe Leu Cys Asn Pro Leu Ile Gln Asn Ile Val Arg Phe Asp
           115                     120                     125
   Thr Glu Phe Ala Phe Lys Thr Asn Ile Ile Asp Thr Ser Lys Asp Leu
       130                     135                     140
   Ile Ile Gly Leu His Gln Val Arg Tyr Lys Ala Thr Lys Glu Arg Pro
   145                     150                     155                     160
   Ser Phe Ser Ser Pro Ile Trp Leu His Lys Asp Asp Glu Pro Val Val
                   165                     170                     175
   Phe Leu His Leu Met Asn Leu Ser Asn Thr Ala Ile Gly Gly Asp Asn
               180                     185                     190
   Leu Ile Ala Asn Ser Pro Arg Glu Ile Asn Gln Phe Ile Ser Leu Lys
           195                     200                     205
   Glu Pro Leu Glu Thr Leu Val Phe Gly Gln Lys Val Phe His Ala Val
       210                     215                     220
   Thr Pro Leu Gly Thr Glu Cys Ser Thr Glu Ala Phe Arg Asp Ile Leu
   225                     230                     235                     240
   Leu Val Thr Phe Ser Tyr Lys Glu Thr Lys
                   245                     250
<210> 14
   <211> 30
   <212> DNA
   <213> Artificial Sequence
<220>
   <223> Primer
<400> 14 ,
   catatgaaaa tgagtggctt tagcatagaa 30
<210> 15
   <211> 30
   <212> DNA
   <213> Artificial Sequence
<220>
   <223> Primer
<400> 15
   ctcgagtttt gtctccttat aagaaaatgt 30
<210> 16
   <211> 753
   <212> DNA
   <213> Bacillus thuringiensis Berliner 1915
<400> 16
   gtggaggttt ttataatgac gtttgttctt agtaaaatga gtggctttag catagaagaa     60
   aaggtacatg aatttgaatc taaaggattc cttgaaattt caaatgaaat ctttttacaa    120
   gaggaagaga atcatcgtct attaacacaa gcacagttag attattataa tttggaagat    180
   gatgcatacg gtgaatgtcg tgctagatct tattcaaggt atataaagta tgttgattca    240
   ccagattata ttttagataa tagtaatgat tacttccaat ctaaagaata taactatgac    300
   gatggcggga aagttagaca gttcaatagc ataaatgata gctttttatg taatccttta    360
   attcaaaata tcgtgcgctt cgatactgaa tttgcattta aaacaaatat aatagataca     420
   agtaaagact taattatagg tttacatcaa gtaagatata aagctactaa agaaagacca    480
   tcttttagtt cacctatttg gttacataaa gatgatgaac cagtagtgtt tttacacctt    540
   atgaatttaa gtaatacagc tattggtgga gataatttaa tagctaattc tcctcgggaa    600
   attaatcagt ttataagttt gaaggagcct ttagaaactt tagtatttgg acaaaaggtc    660
   ttccatgccg taacgccact tggaacagaa tgtagtacgg aggcttttcg tgatatttta    720
   ttagtaacat tttcttataa ggagacaaaa tga                                  753
<210> 17
   <211> 250
   <212> PRT
   <213> Bacillus thuringiensis Berliner 1915
<400> 17
   Met Glu Val Phe Ile Met Thr Phe Val Leu Ser Lys Met Ser Gly Phe
   1               5                   10                  15
   Ser Ile Glu Glu Lys Val His Glu Phe Glu Ser Lys Gly Phe Leu Glu
               20                  25                  30
   Ile Ser Asn Glu Ile Phe Leu Gln Glu Glu Glu Asn His Arg Leu Leu
           35                  40                  45
   Thr Gln Ala Gln Leu Asp Tyr Tyr Asn Leu Glu Asp Asp Ala Tyr Gly
       50                  55                  60
   Glu Cys Arg Ala Arg Ser Tyr Ser Arg Tyr Ile Lys Tyr Val Asp Ser
   65                  70                  75                  80
   Pro Asp Tyr Ile Leu Asp Asn Ser Asn Asp Tyr Phe Gln Ser Lys Glu
                   85                  90                  95
   Tyr Asn Tyr Asp Asp Gly Gly Lys Val Arg Gln Phe Asn Ser Ile Asn
               100                 105                 110
   Asp Ser Phe Leu Cys Asn Pro Leu Ile Gln Asn Ile Val Arg Phe Asp
           115                 120                 125
   Thr Glu Phe Ala Phe Lys Thr Asn Ile Ile Asp Thr Ser Lys Asp Leu
       130                 135                 140
   Ile Ile Gly Leu His Gln Val Arg Tyr Lys Ala Thr Lys Glu Arg Pro
   145                 150                 155                 160
   Ser Phe Ser Ser Pro Ile Trp Leu His Lys Asp Asp Glu Pro Val Val
                   165                 170                 175
   Phe Leu His Leu Met Asn Leu Ser Asn Thr Ala Ile Gly Gly Asp Asn
               180                 185                 190
   Leu Ile Ala Asn Ser Pro Arg Glu Ile Asn Gln Phe Ile Ser Leu Lys
           195                 200                 205
   Glu Pro Leu Glu Thr Leu Val Phe Gly Gln Lys Val Phe His Ala Val
       210                 215                 220
   Thr Pro Leu Gly Thr Glu Cys Ser Thr Glu Ala Phe Arg Asp Ile Leu
   225                 230                 235                 240
   Leu Val Thr Phe Ser Tyr Lys Glu Thr Lys
                   245                 250
<210> 18
   <211> 30
   <212> DNA
   <213> Artificial Sequence
<220>
   <223> Primer
<400> 18
   catatgctaa caacagtttc taataagaca 30
<210> 19
   <211> 30
   <212> DNA
   <213> Artificial Sequence
<220>
   <223> Primer
<400> 19
   ctcgagtttt ggctccttat aagaaaacgt 30
<210> 20
   <211> 741
   <212> DNA
   <213> Bacillus weihenstephanensis KBAB4
<400> 20
   atgctaacaa cagtttctaa taagacaagc agttttgacg tagaacaaaa ggtacatgaa     60
   ttcgaatcaa atggatacat tcaaatcttt aatgatattt ttttacaaga tcaagaagat    120
   caagcattac taacgaaagc acagttagac tactacagct tacaaaacga cgcatatggc    180
   gaatgtcgcg ctagagccta ttcaagatat ataaaatacg ctggttcttc agattatgtt    240
   ctagatacag acaatggata tttccaatct gaagaatata attatgacga tggtgggaaa    300
   attagaaatt tcaacagtat aacagatgaa tttttacata attcattaat tgagaaaatt    360
   gttcgctttg atagtgaatt tgcatctaat acaaatatac ttgatacaag taaggatttg    420
   gttataggtc tacatcaagt aagatataag gcaactagag aaaatccttc ttttagctct    480
   ccaatttggc tacataagga tgatgagccg attgtctttt tacatctcat gaatttaagt    540
   aatacagctc ttggcggaga caatctgatt gcaaacagcc ctagggaaat taacaagctt    600
   attagcttga aggatcccct agaaacttta gtatttggac aaaaggtatt ccacgctgta    660
   acaccactag gaacagagtg taatacagaa gccttacgtg acattttatt agtaacgttt    720
   tcttataagg agccaaaatg a                                               741
<210> 21
   <211> 246
   <212> PRT
   <213> Bacillus weihenstephanensis KBAB4
<400> 21
   Met Leu Thr Thr Val Ser Asn Lys Thr Ser Ser Phe Asp Val Glu Gln
   1               5                   10                  15
   Lys Val His Glu Phe Glu Ser Asn Gly Tyr Ile Gln Ile Phe Asn Asp
               20                  25                  30
   Ile Phe Leu Gln Asp Gln Glu Asp Gln Ala Leu Leu Thr Lys Ala Gln
           35                  40                  45
   Leu Asp Tyr Tyr Ser Leu Gln Asn Asp Ala Tyr Gly Glu Cys Arg Ala
       50                  55                  60
   Arg Ala Tyr Ser Arg Tyr Ile Lys Tyr Ala Gly Ser Ser Asp Tyr Val
   65                  70                  75                  80
   Leu Asp Thr Asp Asn Gly Tyr Phe Gln Ser Glu Glu Tyr Asn Tyr Asp
                   85                  90                  95
   Asp Gly Gly Lys Ile Arg Asn Phe Asn Ser Ile Thr Asp Glu Phe Leu
               100                 105                 110
   His Asn Ser Leu Ile Glu Lys Ile Val Arg Phe Asp Ser Glu Phe Ala
           115                 120                 125
   Ser Asn Thr Asn Ile Leu Asp Thr Ser Lys Asp Leu Val Ile Gly Leu
       130                 135                 140
   His Gln Val Arg Tyr Lys Ala Thr Arg Glu Asn Pro Ser Phe Ser Ser
   145                 150                 155                 160
   Pro Ile Trp Leu His Lys Asp Asp Glu Pro Ile Val Phe Leu His Leu
                   165                 170                 175
   Met Asn Leu Ser Asn Thr Ala Leu Gly Gly Asp Asn Leu Ile Ala Asn
               180                 185                 190
   Ser Pro Arg Glu Ile Asn Lys Leu Ile Ser Leu Lys Asp Pro Leu Glu
           195                 200                 205
   Thr Leu Val Phe Gly Gln Lys Val Phe His Ala Val Thr Pro Leu Gly
       210                 215                 220
   Thr Glu Cys Asn Thr Glu Ala Leu Arg Asp Ile Leu Leu Val Thr Phe
   225                 230                 235                 240
   Ser Tyr Lys Glu Pro Lys
                   245

## Description

### Technical field

The present invention relates to the microbiological industry, and specifically to novel methods for manufacturing 4-hydroxy-L-isoleucine or a salt thereof.

### Background art

4-Hydroxy-L-isoleucine is an amino acid which can be extracted and purified from fenugreek seeds (*Trigonella foenum-graecum L. leguminosae*). 4-Hydroxy-L-isoleucine displays an insulinotropic activity of great interest because its stimulating effect is clearly dependent on plasma glucose concentration in the medium, as demonstrated both in isolated perfused rat pancreas and human pancreatic islets (Sauvaire, Y et al, Diabetes, 47: 206-210, (1998)). Such a glucose dependency is not confirmed on sulfonylureas (Drucker, D. J., Diabetes 47: 159-169, (1998)), the only insulinotropic drug currently used to treat type II diabetes [or non-insulin-dependent diabetes (NIDD) mellitus (NIDDM)], and as a consequence, hypoglycemia remains a common undesirable side effect of sulfonylurea treatment (Jackson, J., and Bessler, R. Drugs, 22: 211-245; 295-320, (1981); Jennings, A. et al. Diabetes Care, 12: 203-208, (1989)). Improvement of glucose tolerance (Am. J. Physiol. Endocrinol., Vol. 287, E463-E471, 2004) is also known. This glucometabolism enhancement activity, and its potential application to pharmaceuticals and health foods, have been reported (Japanese Patent Application Laid-Open No. Hei 6-157302, US2007-000463A1).

4-Hydroxy-L-isoleucine, which is only found in plants, due to its particular insulinotropic action, might be considered as a novel secretagogue with potential interest for the treatment of type II diabetes, a disease characterized by a defective insulin secretion associated with various degrees of insulin resistance (Broca, C. et al, Am. J. Physiol. 277 (Endocrinol. Metab. 40): E617-E623, (1999)).

A method of oxidizing iron, ascorbic acid, 2-oxyglutaric acid, and oxygen-dependent isoleucine by utilizing dioxygenase activity in fenugreek extract has been reported as a method for manufacturing 4-hydroxy-L-isoleucine (Phytochemistry, Vol. 44, No. 4, pp. 563-566, 1997). However, this method is unsatisfactory as a method of manufacturing 4-hydroxy-L-isoleucine because the activity of the enzyme is inhibited by the substrate at isoleucine concentrations of 20 mM and above, the enzyme has not been identified, the enzyme is derived from plant extracts and is not readily obtained in large quantities, and the enzyme is unstable.

An efficient eight-step synthesis of optically pure (2S,3R,4S)-4-hydroxyisoleucine with 39% overall yield has been disclosed. The key steps of this synthesis involve the biotransformation of ethyl 2-methylacetoacetate to ethyl (2S,3S)-2-methyl-3-hydroxy butanoate with *Geotrichum candidum* and an asymmetric Strecker synthesis (Wang, Q. et al, Eur. J. Org. Chem., 834-839 (2002)).

A short six-step chemoenzymatic synthesis of (2S,3R,4S)-4-hydroxyisoleucine with total control of stereochemistry, the last step being the enzymatic resolution by hydrolysis of a N-phenylacetyl lactone derivative using the commercially available penicillin acylase G immobilized on Eupergit C(E-PAC), has also been disclosed (Rolland-Fulcrand, V et al, J. Org. Chem., 873-877 (2004)).

But currently, there have been no reports of cloning any L-isoleucine dioxygenase and producing (2S,3R,4S)-4-hydroxy-L-isoleucine by direct enzymatic hydroxylation of L-isoleucine using the L-isoleucine dioxygenase.

As for production of isoleucine analogues by microorganisms, production of 2-amino-3-keto-4-methylpentanoic acid (AMKP) by *Bacillus* bacteria has been reported (Bioorganic Chemistry, Vol. 6, pp.263-271 (1977)). However, there is no report about isoleucine hydroxylases derived from microorganisms.

Haefele et al., Phytochem. 44(4), 1997, pages 563-566 discloses the biosynthesis of 4-hydroxyisoleucin from isoleucine by a dioxygenase contained in a cell-free extract from fenugreek seedlings, in the presence of various cofactors such as Fe, 2-oxoglutarate, ascorbate and oxygen.

DATABASE UNIPROTKB 1 June 2003, Ivanova et al.: "Genome sequence of Bacillus cereus and comparative analysis with Bacillus anthracis", Database accession no. Q81GX10; DATABASE UNIPROTKB 8 November 2005, Anderson et al.: "Comparative genome analysis of Bacillus cereus group genomes with Bacillus subtilis", Database accession no. Q3EZS9; and DATABASE EMBL 1 January 2006, Ivanova et al.: "Genome sequence of Bacillus cereus and comparative analysis with Bacillus anthracis", Database accession no. AE016877 disclose putative uncharacterized proteins from Bacillus cereus or Bacillus thuringiensis and the corresponding genomic DNA.

### Summary of the invention

An object of the present invention is to provide a method for producing 4-hydroxyisoleucine (used to mean including both free form and a salt form thereof, and may referred to as "4HIL"; hereinafter the same) by using an enzyme derived from a microorganism, which can be prepared in a large amount.

Objects of the present invention include providing a microorganisms having an enzymatic activity for producing 4-hydroxyisoleucine from isoleucine, and a method for producing 4-hydroxyisoleucine from isoleucine based on a hydroxylation reaction using an enzyme derived from a microorganism. The above objects were achieved by finding a microorganisms having an enzymatic activity for producing 4-hydroxyisoleucine from isoleucine.

An object of present invention is to enhance production of (2S,3R,4S)-4-hydroxy-L-isoleucine (used to mean including both free form and a salt form thereof, and may referred to as "(2S,3R,4S)-4HIL"; hereinafter the same), to provide a method for manufacturing (2S,3R,4S)-4-hydroxy-L-isoleucine or a salt thereof by direct enzymatic hydroxylation of L-isoleucine using L-isoleucine dioxygenase or a bacterium having the L-isoleucine dioxygenase activity. As a result of extensive research conducted in consideration of the aforementioned problems, the inventors of the present invention isolated from nature a bacterium having high level of L-isoleucine dioxygenase activity, cloned gene encoding the L-isoleucine dioxygenase and found that the L-isoleucine dioxygenase that may be preferably used in the synthesis of the desired (2S,3R,4S)-4-hydroxy-L-isoleucine, thereby leading to completion of the present invention.

Namely, objects of the present invention include a method for producing (2S,3R,4S)-4-hydroxy-L-isoleucine using the L-isoleucine dioxygenase.
[1] A method for producing 4-hydroxyisoleucine or a salt thereof, which comprises the step of producing 4-hydroxyisoleucine by subjecting isoleucine or a salt thereof to a hydroxylation reaction in the presence of a hydroxylase isolable from a microorganism and producing 4-hydroxyisoleucine from isoleucine,
   wherein said hydroxylase requires Fe²⁺ and 2-oxoglutaric acid as cofactors.
[2] The production method according to [1] above, wherein the microorganism is a microorganism belonging to the genus *Bacillus.*
[3] The production method according to [2] above, wherein the microorganism belonging to the genus Bacillus is a microorganism selected from *Bacillus thuringiensis, Bacillus licheniformis, Bacillus sphaericus, Bacillus cereus,* and *Bacillus weihenstephanensis.*
[4] The production method according to any one of [1] to [3] above, wherein the hydroxylation reaction is performed in the presence of a cell lysate prepared from microbial cells in a logarithmic growth phase.
[5] The production method according to any one of [1] to [4] above, wherein the hydroxylase has the following properties:
   (a) To require oxygen and ascorbic acid as cofactors,
   (b) To have an optimum reaction pH of 5 to 8,
   (c) To have an optimum reaction temperature of 45°C or lower,
   (d) To be inactivated at 50°C or higher,
   (e) To be inhibited by EDTA, Cu²⁺ and Zn²⁺.
[6] A method for manufacturing (2S,3R,4S)-4-hydroxy-L-isoleucine or a salt thereof, comprising the steps of: reacting L-isoleucine in an aqueous solvent in the presence of at least one L-isoleucine dioxygenase or in the presence of a bacterium containing an L-isoleucine dioxygenase,
   wherein the L-isoleucine dioxygenase is selected from the group consisting of:
   (f) a protein comprising the amino acid sequence of SEQ ID No: 2, 8, 13, 17, or 21,
   (g) a protein having an amino acid sequence that contains a substitution, deletion, insertion, addition or inversion of one to 13 amino acid residues in the amino acid sequence of SEQ ID No: 2, 8, 13, 17, or 21 and having L-isoleucine dioxygenase activity, and
   (h) a protein that has at least 95% identity to the amino acid sequence of SEQ ID No: 2, 8, 13, 17, 21 and having L-isoleucine dioxygenase activity; isolating produced (2S,3R,4S)-4-hydroxy-L-isoleucine.
[7] The method according to [6] above, wherein the bacterium is modified to enhance the activity of the L-isoleucine dioxygenase.

### BRIEF DESCRIPTION OF THE DRAWINGS

Fig. 1 shows a graph showing changes over time in production amounts of 4-hydroxyisoleucine and AMKP in culture of the strain 2-e-2.
Fig. 2 shows a graph showing change in turbidity in culture of the strain 2-e-2.
Fig. 3 shows a graph showing activities for producing 4-hydroxyisoleucine and AMKP observed by using resting cells of the strain 2-e-2.
Fig. 4 shows a graph showing 4-hydroxyisoleucine production activities of various samples.
Fig. 5 shows a graph showing pH dependency of the 4-hydroxyisoleucine production activity of a strain 2-e-2 cell lysate.
Fig. 6 shows a graph showing temperature dependency of the 4-hydroxyisoleucine production activity of a strain 2-e-2 cell lysate.
Fig. 7 shows a graph showing temperature stability of the 4-hydroxyisoleucine production activity of a strain 2-e-2 cell lysate.
Fig. 8 shows a graph showing pH dependency of the 4-hydroxyisoleucine production activity of a purified enzyme.
Fig. 9 shows a graph showing temperature dependency of the 4-hydroxyisoleucine production activity of a purified enzyme.
Fig. 10 shows a graph showing temperature stability of the 4-hydroxyisoleucine production activity of a purified enzyme.
Fig. 11 shows a flowchart of the process for producing IDO.
Fig. 12 shows purification of IDO from *Bacillus thuringiensis* strain 2-e-2 (photograph). SDS-PAGE of protein preparation from purification steps is depicted. Lanes: 1 - marker of indicated molecular weights; 2 - crude cell lysate; 3 - ammonium sulphate precipitation; 4 - SEC; 5 -AEC.
Fig. 13 shows MS-identification of IDO from *Bacillus thuringiensis* strain 2-e-2.
Fig. 14 shows putative translation start of *Bacillus thuringiensis* (serovar israelensis, ATCC 35646) RBTH_06809 ORF.
Fig. 15 shows artificial expression modules ofrecombinant pMW119-IDO(Lys, 32/23) plasmids.
Fig. 16 shows physical map of the pMW119-IDO(Lys, 23) plasmid and determined DNA sequence of cloned *BamH*I*-Sac*I fragment harboring IDO structural gene. Spontaneous point mutation in regulatory region marked by grey.
Fig. 17 shows physical map of the pMW119-IDO(Lys, 32) plasmid and determined DNA sequence of cloned *BamH*I*-Sac*I fragment harboring IDO structural gene.
Fig. 18 shows DNA alignment of the structural genes corresponding to IDO(Lys, 23), IDO(Lys, 32) and RBTH_6809 (5'-end truncated). Variable positions marked by grey.
Fig. 19 shows protein alignment of IDO(Lys, 23), IDO(Lys, 32) and RBTH_06890 ORF. Variable positions marked by grey.
Fig. 20 shows Protein alignment of IDO from *Bacillus thuringiensis,* BC1061 from *Bacillus cereus* and conserved hypothetical protein from *Bacillus weihenstephanensis.*

### BEST MODE FOR CARRYING OUT THE INVENTION

In the present specification, 4-hydroxyisoleucine means one type or a mixture of two or more types of diastereomers selected from the group consisting of (2S,3S,4S)-4-hydroxyisoleucine, (2S,3R,4R)-4-hydroxyisoleucine, (2S,3S,4R)-4-hydroxyisoleucine and (2S,3R,4S)-4-hydroxyisoleucine. 4-Hydroxyisoleucine is preferably (2S,3R,4S)- or (2R,3R,4S)-4-hydroxyisoleucine or a mixture thereof, more preferably (2S,3R,4S)-4-hydroxyisoleucine.

In particular, the term "(2S,3R,4S)-4-hydroxy-L-isoleucine" or "(2S,3R,4S)-4HIL" may refer to single chemical compound or mixture containing (2S,3R,4S)-4-hydroxyisoleucine.

The term "bacterium" or "microorganism" as employed in the present Specification includes an enzyme-producing bacteria or microorganisms, a mutant and a genetic recombinant of such bacteria or microorganisms in which the targeted enzymatic activity exists or has been enhanced, and the like.

### <I> Enzymatic activity for producing 4HIL from isoleucine

As the hydroxylase used in the method for producing 4-hydroxyisoleucine of the present invention based on a hydroxylation reaction of isoleucine catalyzed by a hydroxylase, any of crude enzymes as microbial culture, bacterial cells or cell lysate or purified enzymes can be used so long as the chosen material has the enzymatic activity for converting isoleucine to 4-hydroxyisoleucine. Crude enzymes as disrupted cells or purified enzymes are desirable.

Examples of the hydroxylase include oxygenases, dioxygenases and so forth, and dioxygenases are preferred. A hydroxylase that can be isolated from the strain 2-e-2 and have the following properties is more preferred:
(a) To require oxygen, Fe²⁺, ascorbic acid and 2-oxoglutaric acid as cofactors,
(b) To have an optimum reaction pH of 5 to 8,
(c) To have an optimum reaction temperature of 45°C or lower,
(d) To be inactivated at 50°C or higher,
(e) To be inhibited by EDTA, Cu²⁺ and Zn²⁺.

The hydroxylase that can be isolated from the strain 2-e-2 further has the following properties:
(f) To consist of subunits having a molecular weight of 29,000 ± 2,000 as measured by sodium dodecylsulfate polyacrylamide gel electrophoresis,
(g) To have the amino acid sequence of SEQ ID NO: 5 at the N-terminus.

When the hydroxylase requires a cofactor, it is preferable to add or supply the cofactor to the system. Examples of the cofactor for dioxygenase include, for example, Fe²⁺, ascorbic acid and 2-ketoglutaric acid. These cofactors may be added or supplied to the system as a salt thereof when they can form a salt.

As the microorganism, any microorganism can be used so long as a microorganism having an enzymatic activity for converting isoleucine to 4-hydroxyisoleucine under conditions for the hydroxylation reaction is chosen.

Examples of the microorganism used include microorganisms belonging to the genus *Bacillus or Pseudomonas,* mutants or derivatives thereof, and so forth. Further, the microorganism may be a microorganism in which such a hydroxylase is expressed by a gene recombination technique, and which has a 4-hydroxyisoleucine production activity.

Specific examples include *Bacillus thuringiensis* (strain 2-e-2, strain AKU 238, strain NBRC 3958, strain ATCC 35646 etc.), *Bacillus licheniformis* (strain AKU 223, strain IAM 11054 etc.), *Bacillus sphaericus* (strain AKU 227, strain NBRC 3526 etc.), *Bacillus cereus* strain ATCC 14579, and *Bacillus weihenstephanensis* strain KBAB4. The strain 2-e-2 was designated as the AJ110584 strain and deposited at the independent administrative agency, National Institute of Advanced Industrial Science and Technology, International Patent Organism Depositary (Tsukuba Central 6, 1-1, Higashi 1-Chome, Tsukuba-shi, Ibaraki-ken, 305-8566, Japan) under the provisions of the Budapest Treaty on September 27, 2006, and given an accession number of FERM BP-10688.

Among the aforementioned microorganisms, those having a strain name beginning with AKU can be obtained from the Laboratory of Fermentation Physiology and Applied Microbiology, Division of Applied Life Sciences, Graduate School of Agriculture, Kyoto University. Those having a strain name beginning with IAM are maintained at the IAM culture collection, Laboratory of Bioresources, Institute of Molecular and Cellular Biosciences, the University of Tokyo, and can be obtained by using registration numbers. The registration numbers corresponding to the strains are listed in the IAM catalog (IAM Catalogue of Strains Third Edition, 2004). Those having a strain name beginning with NBRC can be obtained from the independent administrative agency, National Institute of Technology and Evaluation (2-5-8, Kazusa Kamatari, Kisarazu-shi, Chiba, 292-0818). Those having a strain name beginning with ATCC can be obtained from the American Type Culture Collection (ATCC) (Postal address: ATCC, P.O. Box 1549, Manassas, VA 20108, 1, United States of America). *Bacillus weihenstephanensis* strain KBAB4 can be obtained from Institut National de la Recherche Agronomique (Postal address: Genetique Microbienne, INRA, Domaine de Vilvert, 79352 Jouy en Josas cedex, France).

The *Bacillus thuringiensis* strain 2-e-2 is a strain newly isolated from the soil in Kyoto city by the inventors of the present invention, and the scientific properties thereof are shown below.

### Taxonomic properties of Bacillus thuringiensis strain 2-e-2

### 1. Phenotype

Cell morphology: Bacillus (size: 1.0 to 1.2 x 2.0 to 3.0 µm)
Gram staining: +
Endospore: +
Attitude to oxygen: Aerobic
Growth temperature: Favorable growth at 20 to 35°C
Optimum pH: pH 7.0 to 7.5

### 2. Molecular phylogenetic analysis based on nucleotide sequence of 16S rDNA

For the 16S rDNA nucleotide sequence of the strain 2-e-2 (SEQ ID NO: 9), the bacterial type strain database (NCIMB Japan, Shizuoka) and international nucleotide sequence databases (GenBank/DDBJ/EMBL) were searched for homology by using BLAST, and 30 most homologous strains were retrieved from each. Then, a molecular phylogenic tree was created by using 16S rDNA nucleotide sequences of the 30 most homologous strains retrieved from the bacteria type strain database and each specimen according to the neighbor-joining method. For the homology search and creation of a simplified molecular phylogenetic tree, DNAsisPro (Hitachi Software Engineering Co., Ltd., Tokyo) was used.

As a result of the homology search of the bacterial type strain database using BLAST, a partial nucleotide sequence of the 16S rDNA of the strain 2-e-2 matched that of the 16S rDNA of the *Bacillus thuringiensis* ATCC 10792 strain with a homology of 100%. As a result of the homology search of GenBank/DDBJ/EMBL, 16S rDNA of the strain 2-e-2 showed a high homology with that of *Bacillus thuringiensis.* Further, as a result of the simplified molecular phylogenetic analysis using 16S rDNA of the strain 2-e-2 and 16S rDNAs of the most homologous 30 strains retrieved from the bacterial type strain database, the strain 2-e-2 formed substantially the same phylogenetic branch as that of the 16S rDNA of *Bacillus thuringiensis,* showing that they are very closely related.

### 3. Results of classification and identification

As a result of simplified morphological observation, the strain 2-e-2 showed common properties of *Bacillus* bacteria, and the results of analysis of partial 16S rDNA sequence also showed that the strain 2-e-2 belonged to *Bacillus thuringiensis.* Since no microorganism that showed AMKP production activity at the same level as the strain 2-e-2 was found, this strain was identified as a novel strain.

The microorganism used in the present invention can be cultured according to a usual culture method. Either a natural medium or a synthetic medium may be used for the culture so long as the medium contains a carbon source, nitrogen source, inorganic salts and so forth that can be assimilated by the microorganism, and the microorganism can be efficiently cultured in the medium.

The carbon source may be one that can be assimilated by the microorganism, and saccharides such as glucose, fructose, sucrose, maltose, starch, starch hydrolysate and molasses, organic acids such as acetic acid, lactic acid and gluconic acid, and alcohols such as ethanol and propanol can be used. As the nitrogen source, ammonia, ammonium salts of various inorganic acids and organic acids such as ammonium sulfate, ammonium chloride, ammonium acetate and ammonium phosphate, other nitrogen-containing compounds, peptone, meat extract, yeast extract, corn steep liquor, casein hydrolysate, soybean meal, soybean meal hydrolysate, various fermenting cells and digestion products thereof, and so forth can be used so long as the microorganism can assimilate them.

As the inorganic salts, potassium phosphate, ammonium sulfate, ammonium chloride, sodium chloride, magnesium sulfate, ferrous sulfate, manganese sulfate, and so forth can be used, so long as the used microorganism can utilize them. In addition, salts of calcium, zinc, boron, copper, cobalt, molybdenum and so forth may be added as trace elements. Further, vitamins such as thiamin and biotin, amino acids such as glutamic acid and aspartic acid, nucleic acid-related substances such as adenine and guanine and so forth may be added as required.

Culture is performed under an aerobic condition such as those obtained by shaking culture or deep aeration stirring culture. The culture temperature is preferably 10 to 37°C, and the culture time is 5 to 40 hours. pH of the culture is maintained at 5.0 to 9.0 during the culture. pH is adjusted by using inorganic or organic acids, alkaline solutions, urea, calcium carbonate, ammonia or the like.

The crude enzyme as disrupted cells, i.e., microbial cell lysate, used in the present invention may be one containing extracellular hydroxylase, and examples thereof include bacterial cells treated with surfactant, organic solvent, or enzyme, those subjected to ultrasonication, mechanical disruption, or solvent treatment, fraction of cellular proteins, solidified product of processed cells, and so forth. The cell lysate is preferably prepared from cells in a logarithmic growth phase.

To prepare a cell lysate, cultured bacterial cells can be washed with an isotonic solution such as physiological saline, and then disrupted by any means, for example, compression disruption using French press, glass beads, ultrasonic disruptor, Manton Gaulin homogenizer, mortar, a combination thereof, or the like. For further efficient disruption, cell membrane surfaces may be physically or chemically treated by freezing treatment, enzymatic treatment, or the like. During the cell disruption, cells are always maintained at a low temperature, and when the cell lysis temperature is raised by the disruption treatment, the temperature may be immediately lowered.

Examples of aqueous medium used for the cell lysate include, but are not limited to, water and buffers such as those of borate, acetate, carbonate, Tris, phosphate, citrate and Good buffer. Further, glycerol, DTT or the like may be added as an enzyme stabilizer, and EDTA, EGTA, PMSF, pepstatin, E-64 or the like may be added as a protease inhibitor. A combination of inhibitors, an inhibitor cocktail or the like may also be added.

As for composition of substrate solution for the 4-hydroxyisoleucine production reaction using the aforementioned cell lysate, 100 mM HEPES buffer (pH 7.0) containing 5 mM isoleucine, 5 mM 2-ketoglutaric acid, 5 mM ascorbic acid and 5 mM Fe²⁺ may be used in a volume of 100 µl, and the reaction is performed at 30°C for 60 minutes. Other than the HEPES buffer, any buffer such as MES buffer and GTA wide range buffer may also be used. After the enzyme is inactivated as required, a centrifugation supernatant fraction of the reaction solution is filtered, and production of 4-hydroxyisoleucine is confirmed by high performance liquid chromatography or TLC.

4-Hydroxyisoleucine may be quantified by any method so long as an analysis system that can separate it from other components is used, and examples of the analysis system include TLC and high performance liquid chromatography. Of these, high performance liquid chromatography is preferred for quantitative analysis, because of the high sensitivity and high separating ability thereof. Examples include the Waters AccQ-Tag™ method, which is an amino acid analysis method, and so forth. By a modified Waters AccQ-Tag™ method (see the examples described later), diastereomers of 4-hydroxyisoleucine can be separated, and naturally occurring 4-hydroxyisoleucine and 2-amino-3-methyl-4-ketopentanoic acid, which is a keto compound formed by oxidation of the hydroxyl group of 4-hydroxyisoleucine, can be separated.

4-Hydroxyisoleucine produced according to the present invention can be isolated by using a commonly used amino acid purification method. For example, by using a combination of ion exchange resin treatment, membrane treatment, crystallization and so forth, 4-hydroxyisoleucine can be isolated from a supernatant of the reaction mixture, from which solids are removed by centrifugation.

In the production method of the present invention, pH for the isoleucine hydroxylation reaction is preferably 5 to 8. As for the composition of the reaction mixture, it is preferred that factors essential for the enzymatic reaction exist in the mixture. When Fe²⁺ is essential as a factor, desirable is a reaction mixture composition unlikely to cause chelation with Fe²⁺ such as those of HEPES buffer, MES buffer and GTA wide range buffer. However, the reaction mixture composition is not limited at all so long as the action of Fe²⁺ is maintained.

In the production method of the present invention, the temperature for the hydroxylation reaction of isoleucine is usually 15 to 30°C, preferably 45°C or lower. The reaction time is usually 5 minutes to 200 hours, although it varies depending on the amount of the enzyme.

As isoleucine used for the hydroxylation reaction, L-isoleucine is preferably used.

Examples of the solvent in which the reaction is performed include aqueous solvents, for example, water, buffers such as those of carbonate, acetate, borate, citrate and Tris, organic solvents, for example, alcohols such as methanol and ethanol, esters such as ethyl acetate, ketones such as acetone, amides such as acetamide, aqueous solvents containing these organic solvents, and so forth. Further, a factor for activating the hydroxylation reaction may be added as required.

The protein concentration of the cell lysate of the microorganism used in the present invention is 0.1 to 50 mg/ml, preferably 0.5 to 20 mg/ml (in terms of cell weight (wet weight)). 4-Hydroxy-L-isoleucine can be produced by adding the cell lysate, substrate and cofactors to an aqueous medium at suitable concentrations and allowing the reaction at a temperature of 45°C or lower, preferably 30°C, and pH 5 to 12, preferably pH 5 to 7.5, for 5 minutes to 120 hours.

### <II> L-Isoleucine dioxygenase and DNA encoding the L-isoleucine dioxygenase, and their uses

The following provides a detailed explanation of [I] L-isoleucine dioxygenase, [II] a process for producing (2S,3R,4S)-4-hydroxy-L-isoleucine using L-isoleucine dioxygenase with reference to the accompanying drawings.

### [I] L-isoleucine dioxygenase

According to research by the inventors of the present invention, bacterial strains were confirmed to contain L-isoleucine dioxygenase having the ability to form (2S,3R,4S)-4HIL in the genus *Bacillus.* The L-isoleucine dioxygenase from microbial cells hereinafter abbreviated as IDO.

As described above, screening of environmental microorganisms provided by the inventors of the present invention revealed unique microbe *Bacillus thuringiensis* strain 2-e-2 possesses activity which could catalyze a reaction in which (2S,3R,4S)-4HIL is directly formed from L-isoleucine (used to mean including both free form and a salt form thereof; hereinafter the same). The inventors of the present invention purified and isolated the novel L-isoleucine dioxygenase from cultivated microbial cells of the microorganisms hereinafter abbreviated as IDO(Lys,23).

Furthermore, the inventors of the present invention determined the N-terminal amino acid sequence of IDO(Lys,23) by purifying dioxygenase derived from *of Bacillus thuringiensis* strain 2-e-2. *Bacillus thuringiensis* strain 2-e-2 was named *Bacillus thuringiensis* AJ110584 and deposited at the International Patent Organism Depositary, National Institute of Advanced Industrial Science and Technology (Central 6, 1-1-1 Higashi, Tsukuba, Ibaraki 305-8566, Japan) on September, 27,2006 and given an accession number of FERM BP-10688 under the provisions of Budapest Treaty.

Furthermore, the inventors of the present invention also synthesized DNA molecules of about 30 base pairs deduced from the amino acid sequences of the IDO(Lys,23), isolated and obtained the entire length of DNAs that encode IDO(Lys,23) using chromosomal DNA from *Bacillus thuringiensis* strain 2-e-2 and L-isoleucine dioxygenase from *Bacillus thuringiensis* (serovar israelensis) strain (ATCC 35646) hereinafter abbreviated as IDO(Lys,32) used as a control, constructed the recombinant plasmids and transformed cells of *E. coli* strain with them. Subsequent analysis of recombinant *E. coli* IDO activity revealed much more higher (5 times higher) activity of IDO(Lys,23) in comparison with IDO(Lys,32) that is consequence of unique mutations in IDO(Lys,23) gene from *Bacillus thuringiensis* strain 2-e-2.

The DNA encoding the IDO(Lys,23) that is identified as mentioned in Example section is shown in SEQ ID No: 1 in the Sequence Listing. Furthermore, the amino acid sequence of IDO(Lys,23) encoded by the nucleotide sequence of SEQ ID NO: 1 is shown in SEQ ID No: 2. SEQ ID NO: 2 is the amino acid sequence of IDO(Lys,23) encoded by the nucleotide sequence of SEQ ID NO: 1. IDO(Lys,23) of SEQ ID NO: 2 possesses the L-isoleucine dioxygenase activity, and catalyze the reaction in which (2S,3R,4S)-4HIL shown in the following formula (I) is directly synthesized from one molecule of L-isoleucine.

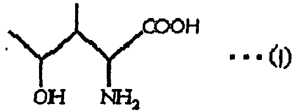

Next, a detailed explanation is provided of (1) a DNA encoding L-isoleucine dioxygenase, (2) properties of L-isoleucine dioxygenase and (3) a process for producing L-isoleucine dioxygenase in that order.

### (1) A DNA Encoding L-isoleucine dioxygenase

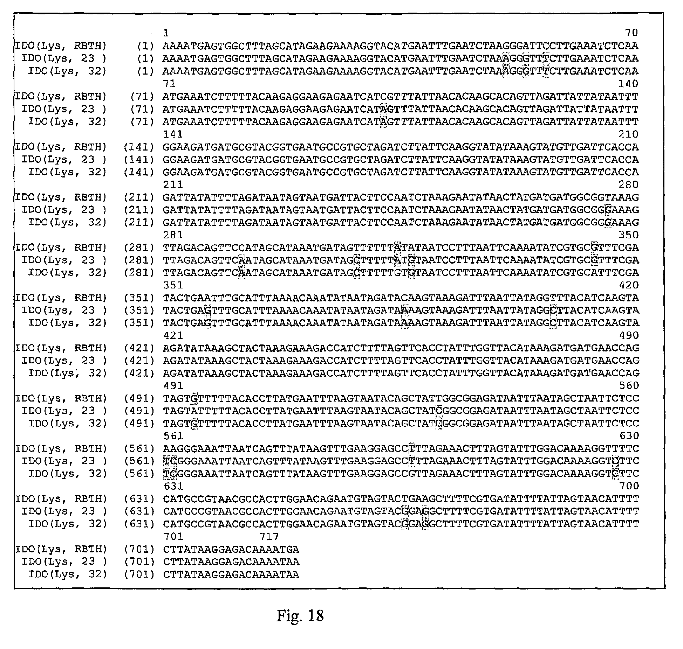

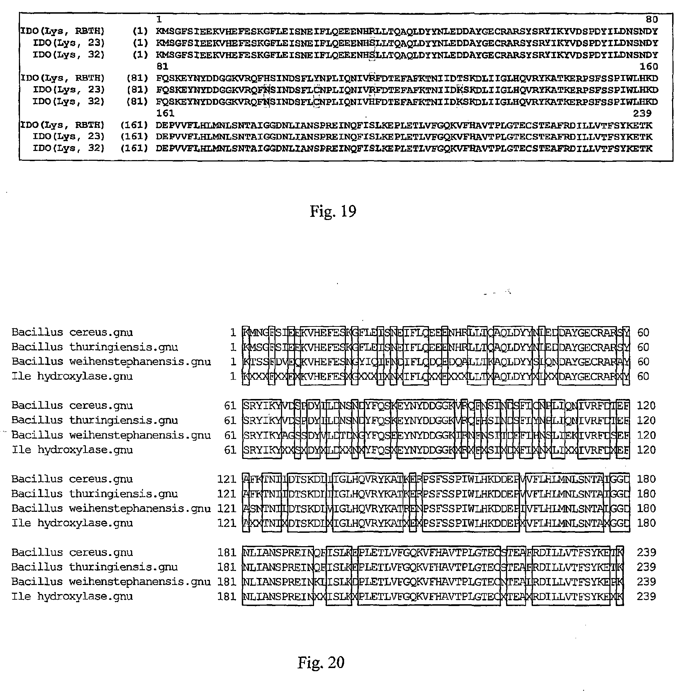

The IDO(Lys,23) gene having the nucleotide sequence of SEQ ID No: 1 was isolated from a chromosomal DNA of *Bacillus thuringiensis* strain 2-e-2 as described in Example section. The nucleotide sequence of SEQ ID No: 1 from *Bacillus thuringiensis* strain 2-e-2 encoding IDO(Lys,23) demonstrates the high level of homology in the nucleotide sequence (Fig. 19) and amino acid sequence (Fig. 18) with non-annotated part of genomic nucleotide sequence from *Bacillus thuringiensis* (serovar israelensis) strain (ATCC 35646), one of which was Submitted 17-JAN-2007 into National Center for Biotechnology Information, NIH, Bethesda, MD 20894, USA (Accession No: AAJM00000000.1, GI:74494335), and the other one was obtained as a result of sequencing genomic DNA encoding IDO(Lys,32) of the same *Bacillus thuringiensis* (serovar israelensis) strain received from Russian National Collection of Industrial Microorganisms (VKPM) stored under accession number VKPM B-197. The nucleotide sequence from *Bacillus thuringiensis* (serovar israelensis) strain VKPM B-197 encoding IDO(Lys,32) is presented in the SEQ ID NO:7.

The following provides an explanation of the method for obtaining an amino acid sequence of IDO from IDO-producing bacteria.

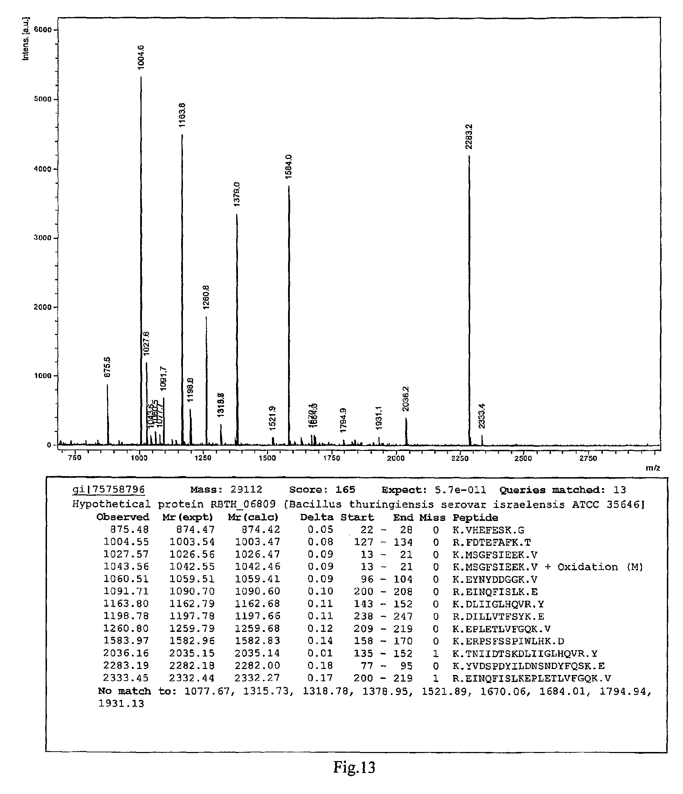

Major protein was recovered from gel and identified by MS-analysis as a putative RBTH_06809 protein from *Bacillus thuringiensis* (serovar israelensis) ATCC 35646 strain (Fig. 13).

Mass spectrometry analysis of the protein sample extracted from SDS-PAGE was carried out. Treatment of gels, trypsinolysis, protein extraction and mass analysis by time-of-flight matrix- assisted laser desorption-ionization (MALDI-TOF) were carried out according to protocols described by Govorun, V.M. et al (The proteome comparative analysis of *Helicobacter pylori* clinical isolates. Biochemistry (Rus), 68, 42-49 (2003)). Protein was identified by the set of its photolytic peptide masses using Peptide Fingerprint option of Mascot software (Matrix Science, USA).

The DNA fragment coding for IDO can be obtained by PCR using appropriate primers designed based on the sequence of from *Bacillus thuringiensis* (serovar israelensis) strain (ATCC 35646).

The procedure employed for PCR is described in publications such as White, T.J. et al., Trends Genet. 5, 185 (1989). The method for isolating a chromosomal DNA, as well as the method for isolating a desired DNA molecule from a gene library using a DNA molecule as a probe, are described in publications such as Molecular Cloning, 3rd edition, Cold Spring Harbor Laboratory Press (2001).

A method for determining the nucleotide sequence of isolated DNA that encodes IDO is described in A Practical Guide to Molecular Cloning, John Wiley & Sons, Inc. (1985). Furthermore, the nucleotide sequence may be determined by using the DNA Sequencer made by Applied Biosystems. A DNA encoding IDO(Lys,23) derived from *Bacillus thuringiensis* strain 2-e-2 is shown in SEQ ID No: 1. The nucleotide sequence from *Bacillus thuringiensis* (serovar israelensis) strain VKPM B-197 that encodes IDO(Lys,32) is shown in SEQ ID No: 7.

A DNA that encodes IDO which catalyzes the reaction in which (2S,3R,4S)-4HIL is formed from L-isoleucine is not only the DNA shown in SEQ ID No: 1. This is because there ought to be differences in nucleotide sequences observed for each species and strain among *Bacillus* species that form IDO which catalyzes the reaction of producing (2S,3R,4S)-4HIL from L-isoleucine.

The DNA not only includes the isolated DNA encoding IDO, but a DNA in which mutations have been artificially added to a DNA that encodes IDO isolated from a chromosomal DNA of a IDO-producing microorganism is also included in the DNA as long as it encodes IDO with activity of catalyzing said reaction. Methods for artificially adding mutations include commonly used methods such as the method for introducing site-specific mutations described in Method, in Enzymol., 154 (1987).

A DNA that hybridizes under stringent conditions with a DNA having a nucleotide sequence complementary to the nucleotide sequence of SEQ ID No: 1, and encodes a protein having IDO activity is also included in the DNA. As used herein, the "stringent conditions" refer to those conditions under which a specific hybrid is formed whereas an non-specific hybrid is not formed. Although it is difficult to numerically express these conditions explicitly, by way of example, mention is made of those conditions under which DNA molecules having higher homology e.g. preferably 70% or more, more preferably 80% or more, still more preferably 90% or more, and particularly preferably 95% or more homology, hybridize with each other, while DNA molecules having lower homology do not hybridize with each other, or those conditions under which hybridization occurs under usual washing conditions in Southern hybridization, that is, at a salt concentration corresponding to 0.1×SSC and 0.1% SDS at 37°C, preferably 0.1×SSC and 0.1% SDS at 60°C, and more preferably 0.1×SSC and 0.1% SDS at 65°C. The length of the probe may be suitably selected, depending on the hybridization conditions, and usually varies from 100 bp to 1 kbp. Furthermore, "L-isoleucine dioxygenase activity" may be sufficient for the activity that synthesizes (2S,3R,4S)-4HIL from L-isoleucine. However, in the case of a nucleotide sequence that hybridizes under stringent conditions with a nucleotide sequence complementary to the nucleotide sequence of SEQ ID No: 1, it preferably retains L-isoleucine dioxygenase activity of 10% or more, preferably 30% or more, more preferably 50% or more, and still more preferably 70% or more, of protein having the amino acid sequence of SEQ ID No: 2 under conditions of 37°C and pH 8.

Furthermore, a DNA encoding a protein which is substantially identical to the IDO encoded by the DNA of SEQ ID No: 1 is also included in the DNA. Namely, the following DNAs are also included in the DNA.
(a) a DNA comprises the nucleotide sequence of SEQ ID No: 1;
(b) a DNA that hybridizes under stringent conditions with a DNA having a nucleotide sequence complementary to the nucleotide sequence of SEQ ID No: 1 and encodes a protein having the L-isoleucine dioxygenase activity;
(c) a DNA that encodes a protein comprising the amino acid sequence of SEQ ID No: 2;
(d) a DNA that encodes a protein having an amino acid sequence that contains a substitution, deletion, insertion, addition or inversion of one or several amino acid residues in the amino acid sequence of SEQ ID No: 2 and having the L-isoleucine dioxygenase activity; and
(e) a DNA that encodes a protein having an amino acid sequence that are at least 70% homologous, preferably at least 80% homologous, more preferably at least 90% homologous and still more preferably at least 95% homologous to the amino acid sequence of SEQ ID NO:2 and having the L-isoleucine dioxygenase activity.

Here, "one or several" refers to the range over which the 3D structure of a protein of amino acid residues or L-isoleucine dioxygenase activity is not significantly impaired, and more specifically, a range of 1 to 78, preferably 1 to 52, more preferably 1 to 26, and still more preferably 1 to 13.

The substitution, deletion, insertion, addition or inversion of one or several amino acid residues should be conservative mutation(s) so that the activity is maintained. The representative conservative mutation is a conservative substitution. Examples of conservative substitutions include substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val.

Furthermore, " L-isoleucine dioxygenase activity" refers to the activity that synthesizes (2S,3R,4S)-4HIL from L-isoleucine described above. However, in the case of an amino acid sequence that contains a substitution, deletion, insertion, addition or inversion of one or several amino acid residues in the amino acid sequence of SEQ ID No: 2, it preferably retains L-isoleucine dioxygenase activity of 10% or more, preferably 30% or more, more preferably 50% or more, and still more preferably 70% or more, of protein having the amino acid sequence of SEQ ID No: 2 under conditions of 30 °C and pH 6.0. The L-isoleucine dioxygenase activity of the IDO can be measured by analysis of (2S,3R,4S)-4HIL formation from L-isoleucine by using high-performance liquid chromatography (HPLC).

Furthermore, a homologue DNA of SEQ ID NO: 1 can be used as a gene encoding L-isoleucine dioxygenase. Wether the homologue DNA encode L-isoleucine dioxygenase or not can be confirmed by measuring L-isoleucine dioxygenase activity of the cell lysate, or cell lysate of the microorganism in which the homologue DNA is overexpressed.

The homologue DNA of SEQ ID NO: 1 can also be prepared from the genome of another *Bacillus* species, for example, *Bacillus cereus, Bacillus weihenstephanensis* as L-isoleucine dioxygenase of present invention.

The alignment of the amino acid sequences of *Bacillus cereus, Bacillus thuringiensis, Bacillus weihenstephanensis* is shown in Figure 20, and conserved sequence between genus of *Bacillus* is shown in SEQ ID NO: 6.

Furthermore, the homologues DNA from another bacterium encoding can be obtained by cloning, based on homologies to the following-listed genes(Table.1) from *Bacillus, Escherichia, Corynebacterium, Arthrobacter, Aspergillus, Pseudomonas, Granulibacter, Methylobacillus, Granulibacter, Acidiphilium, Agrobacterium, Gluconobacter, Caulobacter, Stigmatella, Myxococcus, Polaromonas, Caulobacter Polaromonas, Sphingomonas, Acidovorax, Mycobacterium, Azotobacter, Vibrio, Polynucleobacter, Streptomyces,* or the like. The homologues may be amplified by PCR using, for example, synthetic oligonucleotides shown in SEQ ID NOS: 3 and 4.

**Table.1. List of putative DNA encoding L-isoleucine dioxygenase**

| Gene | Microorganism | Description | Genbank Accession No. |
|---|---|---|---|
| RBTH_06809 | *Bacillus thuringiensis* serovar israelensis ATCC 35646 | Hypothetical protein RBTH_06809 | AAJM01000012.1 |
| | | | GI: 75758796 |
| BC1061 | *Bacillus cereus* ATCC 14579 | hypothetical protein | NC_004722.1 |
| | | | GI:30019216 |
| - | *Bacillus weihenstephanensis* KBAB4 | conserved hypothetical protein | ZP_01182590.1\| |
| | | | GI:89204011 |
| PSPPH_3986 | *Pseudomonas syringae* pv. phaseolicola 1448A | hypothetical protein | NC_005773.3 |
| | | | GI: 71735316 |
| GbCGDNIH1 2096 | *Granulibacter bethesdensis* CGDNIH1 | hypothetical protein | NC_008343.1 |
| | | | GI: 114328760 |
| Mfla_2629 | *Methylobacillus flagellatus* KT | hypothetical protein | NC_007947.1 |
| | | | GI: 91776977 |
| GbCGDNIH1_ 2096 | *Granulibacter bethesdensis* CGDNIH1 | hypothetical protein | NC_008343.1 |
| | | | GI: 114328760 |
| - | *Acidiphilium cryptum* JF-5 | conserved hypothetical protein | ZP_01144511.1 |
| | | | GI: \|88939060 |
| - | *Agrobacterium vitis* | hypothetical protein | ABG82019.1 |
| | | | GI:110671820 |
| GOX1674 | *Gluconobacter oxydans* 621H | hypothetical protein | YP:192070.1 |
| | | | GI:58040106 |
| - | *Caulobacter sp.* K31 | conserved hypothetical protein | ZP:01420729.1 |
| | | | GI:113934829 |
| - | *Stigmatella aurantiaca* DW4/3-1 | conserved hypothetical protein | ZP_01462001.1 |
| | | | GI:115374724 |
| MXAN_6813 | *Myxococcus xanthus* DK 1622 | hypothetical protein | YP:634930. |
| | | | GI:108759113 |
| Bpro_0594 | *Polaromonas* sp. JS666 | hypothetical protein | YP:547452.1 |
| | | | GI:91786500 |
| CC3057 | *Caulobacter crescentus* CB1 | hypothetical protein | NP_421851.1 |
| | | | GI:16127287 |
| - | *Polaromonas naphthalenivorans* CJ2 | similar to Uncharacterized protein conserved in bacteria | ZP_01022090.1 |
| | | | GI:84714798 |
| *-* | *Sphingomonas* sp. SKA58 | putative phage repressor | ZP_01302473 |
| | | | GI:94495894 |
| | *Acidovorax* sp. JS42 | conserved hypothetical protein | ZP_01384166.1 |
| | | | GI:110595841 |
| | *Mycobacterium* sp. JLS | conserved hypothetical protein | ZP_01276363.1 |
| | | | GI: 92907583 |
| | *Azotobacter vinelandii* AvOP | similar to Uncharacterized protein conserved in bacteria | ZP_00417642.1 |
| | | | GI 67156016 |
| VV21380 | *Vibrio vulnificus* CMCP6 | hypothetical protein | NP_763273.1 |
| | | | GI 27367746 |
| VVA0217 | *Vibrio vulnificus* YJ016 | hypothetical protein | NP_936273.1 |
| | | | GI 37675877 |
| - | *Polynucleobacter* sp. QLW-P1DMWA-1 | conserved hypothetical protein | ZP_01493168.1 |
| | | | GI 116268923 |
| AF484556_24 | *Streptomyces atroolivaceus* | conserved hypothetical protein | AAN85502.1 |
| | | | GI:26541515 |

### (2) Properties of IDO

Next, an explanation is provided of the properties of purified L-isoleucine dioxygenase derived from *Bacillus thuringiensis* strain 2-e-2 (IDO(Lys,23)).

The IDO(Lys,23) has the amino acid sequence of SEQ ID No: 2 as is clearly determined by the previously described gene isolation and analysis. However, the protein used in the present invention includes a protein having an amino acid sequence that contains a substitution, deletion, insertion, addition or inversion of one or several amino acid residues in the amino acid sequence of SEQ ID No: 2, 8, 13, 17, or 21, which also has L-isoleucine dioxygenase activity.

Namely, the IDO includes the following proteins:
(f) a protein comprises the amino acid sequence of SEQ ID No: 2, 8, 13, 17, or 21;
(g) a protein having an amino acid sequence that contains a substitution, deletion, insertion, addition or inversion of one or a several amino acid residues in the amino acid sequence of SEQ ID No: 2, 8, 13, 17, or 21 and having L-isoleucine dioxygenase activity; and
(h) a protein that are at least 70% homologous, preferably at least 80% homologous, more preferably at least 90% homologous and still more preferably at least 95% homologous to the amino acid sequence of SEQ ID NO: 2, 8, 13, 17, or 21 and having L-isoleucine dioxygenase activity.

Here, the definitions of "several" and "L-isoleucine dioxygenase activity" are the same as defined in section (1), DNA Encoding L-isoleucine dioxygenase.

The IDO catalyzes the reaction that synthesizes (2S,3R,4S)-4HIL by hydroxylation reaction from L-isoleucine.

The L-isoleucine dioxygenase activity of the IDO may be measured by analysis of (2S,3R,4S)-4HIL formation from L-isoleucine by using high-performance liquid chromatography (HPLC).

The IDO is able to catalyze the reaction that synthesizes (2S,3R,4S)-4HIL by hydroxylation reaction from L-isoleucine. In hydroxylation reaction catalyzed by dioxygenases, one atom of molecular oxygen is incorporated into L-isoleucine, while the other oxygen atom is incorporated in other oxygen acceptor, for example α-ketoglutarate, resulting in the formation of (2S,3R,4S)-4HIL and succinate with release of carbon dioxide. Dioxygenases are capable of hydroxylating an aliphatic carbon chain in a stereospecific way. Although one example of plant enzymes capable of catalyzing hydroxylation reaction of L-isoleucine as a substrate have been reported thus far consisting of L-isoleucine dioxygenase derived from fenugreek extract (Phytochemistry, Vol. 44, No. 4, pp. 563-566, 1997). However, this method is unsatisfactory as a method of manufacturing 4-hydroxy-L-isoleucine because the activity of the enzyme is inhibited by the substrate at isoleucine concentrations of 20 mM and above, the enzyme has not been identified, the enzyme is derived from plant extracts and is not readily obtained in large quantities, and the enzyme is unstable.

Next, the following provides a description of the enzymatic properties investigated for purified IDO(Lys,23).

IDO(Lys,23) catalyzes the reaction that forms (2S,3R,4S)-4HIL represented by the following general formula (I): 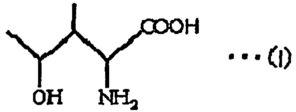 from L-isoleucine in reaction shown below:

**L-isoleucine + α-ketoglutarate + O₂ → 4HIL + succinate + CO₂**

Thus, a process of producing (2S,3R,4S)-4HIL from L-isoleucine by using IDO(Lys,23) also belongs to the present invention.

Furthermore, the activity of DO(Lys,23) strictly depends on the bivalent cation Fe²⁺ and is completely blocked in the presence of EDTA. IDO(Lys,23) is able to catalyze transfer one of the oxygen atom to L-isoleucine and another oxygen atom to α-ketoglutarate as acceptor molecule. So, IDO(Lys,23) may belong to the α -ketoglutamate dependent dioxygenase.

Since the molecular weight of IDO(Lys,23) per subunit as measured by SDS-PAGE is about 29±2.0 kDa. Therefore, the protein used in the present invention also includes a protein as defined by following characteristics:
(A) has an activity that catalyze a reaction of producing (2S,3R,4S)-4HIL from L-isoleucine and α-ketoglutarate;
(B) the activity is dependent on bivalent cation including Fe²⁺ and
(C) the molecular weight per subunit as measured by SDS-PAGE is about 29±2.0 kDa.

The IDO(Lys,32) from *Bacillus thuringiensis* (serovar israelensis) strain VKPM B-197 has the amino acid sequence of SEQ ID NO: 8.

### (3) Process for producing L-isoleucine dioxygenase

Next, an explanation is provided for the process of producing the IDO. There are two ways to produce the IDO used in the present invention. These consist of (i) a process of cultivating an IDO-producing microorganism to form and accumulate IDO, and (ii) a process of preparing a transformant to form IDO by a recombinant DNA technology and cultivating the transformant to accumulate IDO.

### (i) Process for Forming and Accumulating IDO by Microbial Cultivation

Examples of microorganisms serving as acquisition sources of IDO in a process for forming and accumulating IDO by cultivating IDO-producing microorganisms include microorganisms belonging to the genus *Escherichia, Pseudomonas, Corynebacterium, Arthrobacter, Aspergillus* or *Bacillus.*

Any microorganism belonging to the genus *Bacillus, Escherichia, Corynebacterium, Arthrobacter, Aspergillus, Pseudomonas, Granulibacter, Methylobacillus, Granulibacter, Acidiphilium, Agrobacterium, Gluconobacter, Caulobacter, Stigmatella, Myxococcus, Polaromonas, Caulobacter, Polaromonas, Sphingomonus, Acidovorax, Mycobacterium, Azotobacter, Vibrio, Polynucleobacter, Streptomyces* may be used in the present invention provided they are microorganisms that form IDO which catalyzes a reaction that synthesizes (2S,3R,4S)-4HIL from L-isoleucine and α-ketoglutarate, and preferable microorganisms include *Bacillus thuringiensis* strain 2-e-2 and *Bacillus thuringiensis* (serovar israelensis; ATCC 35646) strain. Among these, *Bacillus thuringiensis* strain 2-e-2 is particularly preferable.

Although the microorganism serving as the acquisition source of IDO may be cultivated in any form such as liquid cultivation and solid cultivation, an industrially advantageous method is deep-aerated stir cultivation. Carbon sources, nitrogen sources, inorganic salts, and other trace nutrient elements commonly used in microbial cultivating may be used as nutrient elements of nutritive media. All nutrient sources may be used so long as those are usable by the microbial strain being used.

Culturing is conducted under aerobic conditions by shake culturing, deep ventilation stir culturing, or the like. The cultivating temperature may be sufficient within a range in which the microorganisms grow and IDO is produced. Thus, although the conditions are not strict, the cultivating temperature is normally 10 to 50°C and preferably 15 to 42 °C. The cultivating time varies according to other cultivating conditions. For example, the microorganisms may be cultivated until the greatest amount of IDO is produced, and this is normally about 5 hours to 7 days, and preferably about 10 hours to 96 hours.

Following cultivation, the microbial cells are recovered by centrifugation (e.g., 10,000xg for 10 minutes). Since the majority of the IDO is present in the cells, the IDO is solubilized by disrupting or lysing the microbial cells. Ultrasonic disrupting, French press disrupting or glass bead disrupting may be used to disrupt the microbial cells. In the case of lysing the cells, a method that uses egg white lysozyme, peptidase treatment, or a suitable combination thereof is adopted.

When IDO derived from an IDO-producing microorganism is purified, although the IDO is purified by using an enzyme solubilizing solution for the starting material, if undisrupted or unlysed residue remains, re-centrifuging the solubilization solution and removing any residue that precipitates is advantageous to purification.

All commonly used methods for purifying ordinary enzymes may be employed to purify the IDO, examples of which include ammonium sulfate precipitation, gel filtration chromatography, ion exchange chromatography, hydrophobic interaction chromatography and hydroxyapatite chromatography. As a result, an IDO-containing fraction with higher specific activity may be obtained having higher specific activity.

### (ii) Production Process Using Recombinant DNA Technology

Next, an explanation is provided for a process for producing IDO using a recombinant DNA technology. There are numerous known examples of producing useful proteins such as enzymes and physiologically active substances using a recombinant DNA technology, and the use of recombinant DNA technology enables mass production of useful proteins present only in trace amounts in nature.

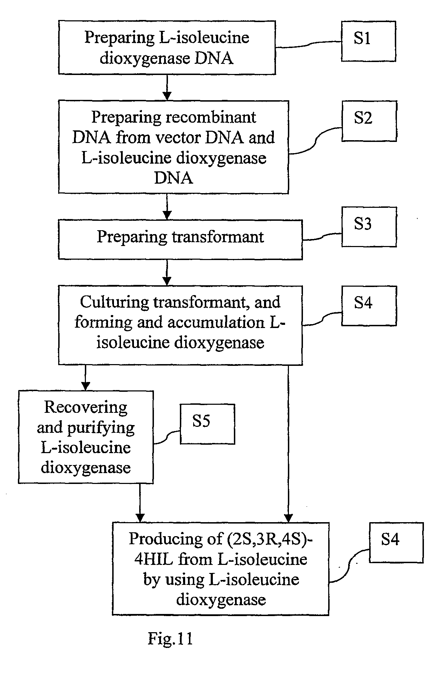

Fig. 11 is a flowchart of a process for producing the IDO used in the present invention.

First, a DNA is prepared that encodes the IDO (Step S1).

Next, the prepared DNA is ligated with a vector DNA to produce a recombinant DNA (Step S2), and cells are transformed by the recombinant DNA to produce a transformant (Step S3). Continuing, the transformant is cultivated in a medium, and the IDO is allowed to form and accumulate in any one of the medium and cells or both (Step S4).

Subsequently, the process proceeds to Step S5 where purified IDO is produced by recovering and purifying the enzyme.

The desired (2S,3R,4S)-4HIL may be produced in a large amount by using the purified IDO produced at Step S5 or any of the medium and cells or both in which IDO has accumulated at Step S4 in a hydroxylation reaction(Step S6).

The DNA that is ligated with the vector DNA may allow expression of the IDO.

Here, examples of IDO genes ligated into the vector DNA include the previously described DNA as in [I].

In the case of large scale protein production using recombinant DNA technology, cells such as bacterial *cells, Actinomyces* cells, yeast cells, mold cells, plant cells and animal cells may be used for the host cells that are transformed. Examples of bacterial cells for which host-vector systems have been developed include *Escherichia* species, *Pseudomonas* species, *Corynebacterium* species, *Arthrobacter* species, *Aspergillus* species and *Bacillus* species, and preferably *Escherichia coli* or *Corynebacterium glutamicum* is used. This is because there is numerous knowledge regarding technologies for mass production of protein using *Escherichia coli,Corynebacterium glutamicum* or *Bacillus* bacterium. The following provides an explanation of a process for producing L-isoleucine dioxygenase using transformed *E*. *coli.* The following method for *E*. *coli* can also be applied to *Corynebacterium glutamicum* or *Bacillus* bacterium.

A promoter normally used for heterogeneous protein production in *E*. *coli* may be used for the promoter that expresses a DNA encoding IDO, examples of which include powerful promoters such as T7 promoter, trp promoter, lac promoter, tac promoter and PL promoter.

In order to produce IDO in the form of a fused protein inclusion body, a gene that encodes another protein, preferably a hydrophilic peptide, is ligated either upstream or downstream of the IDO gene with a fused protein gene. The gene that encodes another protein may be a gene that increases the amount of fused protein accumulated and enhances the solubility of the fused protein following the denaturation and regeneration steps, examples of candidates for which include T7 gene 10, β-galactosidase gene, dehydrofolate reductase gene, interferon-γ gene, interleukin-2 gene and prochymosin gene.

When ligating these genes with a gene that encodes IDO, the codon reading frames are made to match. The genes may either be ligated in a suitable restriction enzyme site or using a synthetic DNA of an appropriate sequence.

In order to increase the amount produced, it is preferable to couple a transcription terminating sequence in the form of a terminator downstream from the fused protein gene. Examples of this terminator include T7 terminator, fd phage terminator, T4 terminator, tetracycline resistance gene terminator, and *E. coli trpA* gene terminator.

Multi-copy vectors are preferable for the vector used to introduce a gene that encodes IDO or a fused protein of IDO with another protein into *E. coli,* examples of which include plasmids having a replication starting point derived from Col E1 such as pUC plasmids, pBR322 plasmids or their derivatives. A "derivative" here refers to that has undergone alteration of a plasmid by base substitution, deletion, insertion, addition or inversion. The alteration referred to here includes alteration caused by mutagenic treatment using a mutagen or UV irradiation or by spontaneous mutation or random mutation.

It is preferable that the vector has a marker such as ampicillin resistance gene in order to select the transformant. Examples of such plasmids include commercially, available expression vectors having a powerful promoter (such as pUC (Takara), pPROK (Clontech), and pKK233-2 (Clontech)).

A recombinant DNA is obtainable from ligating a DNA fragment, in which a promoter, a gene encoding IDO or fused protein consisting of IDO and another protein, and a terminator are ligated in that order, with a vector DNA.

When *E. coli* is transformed using the recombinant DNA and that *E*. *coli* is then cultivated, IDO or a fused protein of IDO with another protein is expressed and produced. A strain that is normally used for expression of heterogeneous genes may be used for the transformed host, and *E. coli* strain JM109 (DE3) and *E. coli* strain JM109 are particularly preferable. The transformation method and method for selecting the transformant are described in, for example, Molecular Cloning, 3rd edition, Cold Spring Harbor Laboratory Press (2001).

In the case of expressing as fused protein, the IDO may be cut out using a restricting protease such as blood coagulation factor Xa or kallikrein that recognizes sequences not existing in IDO as the recognition sequence.

A medium normally used for cultivating *E. coli* may be used for the production medium, examples of which include M9-casamino acid medium and LB medium. Furthermore, conditions of cultivating and production induction may be appropriately selected according to the type of the marker and promoter of the vector used, and type of host microorganism used.

The following method may be used to recover the IDO or fused protein of IDO with another protein. If the IDO or its fused protein is solubilized within microbial cells, then it may be used in the form of a crude enzyme solution after recovering the microbial cells and disrupting or lysing the recovered cells. Furthermore, the IDO or its fused protein may also be used after purifying by precipitation, filtration, column chromatography or other common technique as necessary. In this case, a purification method may also be used that uses an antibody of the IDO or its fused protein.

When a protein inclusion body is formed, it is solubilized with a denaturant. Although it may be solubilized together with microbial cell protein, in consideration of the subsequent purification procedure, it is preferable to take out the inclusion body and then solubilize it. A method known in the prior art may be used to recover the inclusion body from the microbial cell. For example, the inclusion body may be recovered by disrupting the microbial cell followed by centrifugal separation. Examples of denaturants that solubilize protein inclusion bodies include guanidine hydrochloride (e.g., 6 M, pH 5-8) and urea (e.g., 8 M).

The protein inclusion body may be regenerated as active protein by removing these denaturants by treatment such as dialysis. Dialysis solutions such as Tris-HCl buffer or phosphate buffer may be used for dialysis, and the concentration may be from 20 mM to 0.5 M, and the pH may be from pH 5 to pH 8.

The protein concentration during the regeneration step is preferably held to about 500 µg/ml or less. In order to prevent the regenerated IDO from undergoing self-crosslinking, the dialysis temperature is preferably 5°C or lower. Furthermore, activity may also be expected to be restored by other methods used to remove denaturant such as dilution and ultrafiltration in addition to the aforementioned dialysis.

When the IDO gene is derived from bacteria belonging to the genus *Bacillus,* the IDO may be expressed and produced by using the bacteria of *Escherichia, Pseudomonas, Corynebacterium, Arthrobacter, Aspergillus* or *Bacillus* genus as a host in one preferable mode.

The copy number of the gene may be increased by inserting a gene into a multi-copy vector, followed by introducing the vector into a microorganism. Vectors which can be used include *E. coli* plasmid vectors such as pMW118, pBR322, pUC19, pBluescript KS+, pACYC177, pACYC184, pAYC32, pMW119, pET22b, *E. coli - B. subtilis* shuttle vectors such as pHY300PLK, pGK12, pLF14, pLF22 or the like, phage vectors such as 11059, 1BF101, M13mp9, Mu phage (Japanese Patent Application Laid-Open No. 2-109985), or the like, and transposons (Berg, D.E. and Berg, C.M., Bio/Technol., 1, 417 (1983)), such as Mu, Tn10, Tn5, or the like. It is also possible to increase the copy number of a gene by integrating the gene into a chromosome by homologous recombination utilizing a plasmid, or the like. Examples of host cells and expression system in this case may include a report on a recombinant expression method in *Arthrobacter sp.* by Shaw P. C. et al. (J Gen Micobiol. 134 (1988) p.903-911), a report on a recombinant expression method in *Arthrobacter nicotinovorans* by Sandu C. et al. (Appl Environ Microbiol. 71 (2005) p8920-8924) and a report on a recombinant expression method in *Arthrobacter sp.* by Morikawa, M. et al. (Appl Microbiol Biotechnol., 42 (1994), p.300-303). Also, there are several reports that expression system developed for Coryneform bacteria is also usable for *Arthrobacter* species (Sandu C. et al.). However, the *Bacillus* species bacteria used as host cells and usable expression system for *Bacillus* IDO are not limited to those recited herein.

### [II] Method for producing (2S,3R,4S)-4-hydroxy-L-isoleucine

Method for producing (2S,3R,4S)-4-hydroxy-L-isoleucine ((2S,3R,4S)-4HIL) represented by the general formula (I) of the present invention comprises one step reaction of direct enzymatic hydroxylation of the L-isoleucine to produce the (2S,3R,4S)-4HIL represented by the following reaction:

**L-isoleucine + α-ketoglutarate + O₂ → 4HIL + succinate + CO₂**

wherein the reaction is performed in the presence of L-isoleucine as acceptor molecule of one of the oxygen atom, α-ketoglutarate as acceptor molecule of another oxygen atom, one oxygen molecule as a donor of two oxygen atoms and IDO that catalyzes the reaction.

In the present invention, "enzymatic hydroxylation" means a hydroxylation reaction which is carried out by an IDO enzyme. Particularly a bacterial IDO is preferred.

There are no particular limitations on the IDO that catalyzes the reaction, and any protein may be used as long as the protein is capable of catalyzing a reaction hydroxylation of L-isoleucine in the presence of α-ketoglutarate and oxygen.

A preferable example of such an IDO is IDO explained in section [1] describing IDO. In the method of the present invention, IDO may be used in any form such as a bacterium (including a culture, bacterial cells, or treated cells), a purified enzyme, or a crude enzyme, so long as it incorporates the above IDO which catalyze the reaction of producing the aforementioned (2S,3R,4S)-4HIL. When a bacterium is used as a source of an IDO, both (1) a bacterium which naturally produce the IDO, such as microorganisms belonging to the genus *Bacillus,* and (2) a recombinant microorganism transformed by a recombinant DNA as described in the section [1] are usable to accumulate the IDO by cultivating such microorganisms.

Protein sequence which is used for the characterization of IDO is categorized as L-isoleucine dioxygenase (SEQ ID NO: 2, SEQ ID NO:8 Table 1).

L-Isoleucine dioxygenase used in the present invention also includes a protein as defined by following characteristics:
(A) has an activity that catalyze a reaction of producing (2S,3R,4S)-4HIL from L-isoleucine and α-ketoglutarate;
(B) the activity is dependent on bivalent cation including Fe²⁺ and
(C) the molecular weight per subunit as measured by SDS-PAGE is about 29±2.0 kDa.

For example, in the step of producing (2S,3R,4S)-4HIL using IDO-producing bacterium or bacterial cells that have been transformed by a recombinant DNA, the substrate may be added directly to the culture media while cultivating, or may be used in the form of bacterial cells or washed bacterial cells that have been separated from the culture. Furthermore, treated bacterial cells that have been disrupted or lysed may be used directly, or the IDO may be recovered from the treated bacterial cells and used as a crude enzyme solution, or used after purifying the enzyme. Namely, as long as it is in the form of a fraction that has IDO, it may be used in the process for producing a (2S,3R,4S)-4HIL of the present invention.

In order to perform an hydroxylation reaction using IDO, a reaction solution containing L-isoleucine, α-ketoglutarate and a protein or IDO-containing composition that catalyzes the reaction is adjusted to a suitable temperature of 20 to 50°C and allowing to stand undisturbed, shaking or stirring for 30 minutes to 5 days while maintaining at pH 5 to 12.

The reaction velocity may also be improved by adding a bivalent cation such as Fe²⁺ to the reaction mixture.

When adding these bivalent cations to the reaction solution, although any salt may be used provided it does not hinder the reaction, FeSO₄, and so forth may be used preferably. The concentrations of these bivalent cations may be determined by simple preliminary studies conducted by a person with ordinary skill in the art. These bivalent cations may be added within the range of 0.01 mM to 50 mM, preferably 0.1 mM to 25 mM.

Oxygen goes into reaction from air as a result of agitation with fixed regime of culture volume during cultivation.

The (2S,3R,4S)-4HIL of general formula (I) that is formed in the reaction mixture may be either separated or purified according to known techniques, or further used as it is especially when the reaction is performed with a recombinant microorganism expressing IDO.

Examples of separation and purification method may include a method in which the (2S,3R,4S)-4HIL is contacted with an ion exchange resin to adsorb basic amino acids followed by elution and crystallization, and a method in which the product obtained by elution is discolored and filtrated with activated charcoal followed by crystallization to obtain (2S,3R,4S)-4HIL.

Non-annotated genes encoding IDO from other microorganisms can be identified by homology to known IDO genes, followed by evaluation of the activity of proteins encoded by these genes.

Homology between two amino acid sequences can be determined using well-known methods, for example, the computer program BLAST 2.0, which calculates three parameters: score, identity, and similarity.

Therefore, the DNA fragment from *Bacillus thuringiensis* strain 2-e-2 and *Bacillus thuringiensis* (serovar israelensis; ATCC 35646) strain encoding full-length IDO can be obtained by PCR (polymerase chain reaction; refer to White, T.J. et al., Trends Genet., 5, 185 (1989)) utilizing primers prepared based on the known fragment of amino acid and nucleotide sequence. DNA fragment encoding IDO from other microorganisms can be obtained in a similar manner.

Since there may be some differences in DNA sequences between bacterial strains, the above-described fragments encoding IDO to be used are not limited to the nucleotide sequences shown in SEQ ID NO: 1, 7, 12, 16, 20, Fig. 18 or Table 1, but may also include nucleotide sequences similar to those shown in SEQ ID NO: 1, 7, 12, 16, 20, Fig. 18 or Table 1. Therefore, the protein variants encoded by the above-described genes may have a similarity of not less than 80%, preferably not less than 90%, and most preferably not less than 95%, with respect to the entire amino acid sequences shown in SEQ ID NOS. 2, 8, 13, 17, 21, Fig. 19 or Table 1, as long as the abilities of the proteins to catalyze the said reactions are maintained.

Moreover, the above-described DNA fragments may be represented by variants which can hybridize under stringent conditions with the nucleotide sequences shown in SEQ ID NOS: 1, 7, 12, 16, 20, Fig. 18 or Table 1, or with probes prepared based on these nucleotide sequences, provided that they encode functional proteins. "Stringent conditions" used here is the same as aforementioned section, [1] (1) A DNA Encoding IDO.

Examples of forms of the treated bacterial cells employed in the present invention include dried bacterial mass, freeze-dried bacterial mass, products treated with surfactants or organic solvents, enzyme-treated products, ultrasound-treated products, mechanically ground products, solvent-treated products, protein fractions of bacterial mass, immobilized products of bacterial mass and processed bacterial mass.

IDO may be prepared separately as described above and added into the reaction solution. A bacterium (host cell) that expresses the DNA encoding IDO may be prepared by transfection of the expression vector functionally containing the DNA encoding the IDO in the form capable of expression in the host cell with those activities. Further, the host cells having enhanced activities of the IDO by increasing the expression of the genes encoding IDO are preferably used.

The phrase "increasing the expression of the gene" means that the expression of the gene is higher than that of a non-modified strain, for example, a wild-type strain. Examples of such modifications include increasing the copy number of expressed gene(s) per cell, increasing the expression level of the gene(s), and so forth. The quantity of the copy number of an expressed gene is measured, for example, by restricting the chromosomal DNA followed by Southern blotting using a probe based on the gene sequence, fluorescence in situ hybridization (FISH), and the like. The level of gene expression can be measured by various known methods including Northern blotting, quantitative RT-PCR, and the like. The amount of the protein encoded by the gene can be measured by known methods including SDS-PAGE followed by immunoblotting assay (Western blotting analysis), and the like.

"Transformation of a bacterium with DNA encoding a protein" means introduction of the DNA into a bacterium, for example, by conventional methods. Transformation of this DNA will result in an increase in expression of the gene encoding the protein used in the present invention, and will enhance the activity of the protein in the bacterial cells. Methods of transformation include any known methods that have hitherto been reported. For example, a method of treating recipient cells with calcium chloride so as to increase permeability of the cells to DNA has been reported for *Escherichia coli* K-12 (Mandel, M. and Higa, A., J. Mol. Biol., 53, 159 (1970)) and may be used.

Methods of enhancing gene expression include increasing the gene copy number. Introducing a gene into a vector that is able to function in a bacterium increases the copy number of the gene. For such purposes multi-copy vectors can be preferably used. The multi-copy vector is exemplified by pBR322, pMW119, pUC 19, pET22b, or the like.

Enhancement of gene expression may also be achieved by introduction of multiple copies of the gene into a bacterial chromosome by, for example, homologous recombination, Mu integration, or the like. For example, one act of Mu integration allows for introduction of up to 3 copies of the gene into a bacterial chromosome.

Increasing the copy number of the gene can also be achieved by introducing multiple copies of the gene into the chromosomal DNA of the bacterium. In order to introduce multiple copies of the gene into a bacterial chromosome, homologous recombination is carried out using a sequence which exists in multiple copies as targets in the chromosomal DNA. Sequences having multiple copies in the chromosomal DNA include, but are not limited to repetitive DNA, or inverted repeats existing at the end of a transposable element. Also, as disclosed in US patent No. 5,595,889, it is possible to incorporate the gene into a transposon, and allow it to be transferred to introduce multiple copies of the gene into the chromosomal DNA.

Enhancing gene expression may also be achieved by placing the DNA under the control of a potent promoter. For example, the Ptac promoter, the lac promoter, the trp promoter, the trc promoter, the P_{R}, or the P_{L} promoter of lambda phage are all known to be potent promoters. The use of a potent promoter can be combined with multiplication of gene copies.

Alternatively, the effect of a promoter can be enhanced by, for example, introducing a mutation into the promoter to increase the transcription level of a gene located downstream of the promoter. Furthermore, it is known that substitution of several nucleotides in the spacer between ribosome binding site (RBS) and the start codon, especially the sequences immediately upstream of the start codon, profoundly affect the mRNA translatability. For example, a 20-fold range in the expression levels was found, depending on the nature of the three nucleotides preceding the start codon (Gold et al., Annu. Rev. Microbiol., 35, 365-403, 1981; Hui et al., EMBO J., 3, 623-629, 1984). Previously, it was shown that the rhtA23 mutation is an A-for-G substitution at the -1 position relative to the ATG start codon (ABSTRACTS of 17th International Congress of Biochemistry and Molecular Biology in conjugation with 1997 Annual Meeting of the American Society for Biochemistry and Molecular Biology, San Francisco, California August 24-29, 1997, abstract No. 457).

Moreover, it is also possible to introduce a nucleotide substitution into a promoter region of the gene on the bacterial chromosome, which results in stronger promoter function. The alteration of the expression control sequence can be performed, for example, in the same manner as the gene substitution using a temperature-sensitive plasmid, as disclosed in International Publication WO 00/18935 and Japanese PatentApplication Laid-Open No. 1-215280.

Methods for preparation of plasmid DNA include, but are not limited to digestion and ligation of DNA, transformation, selection of an oligonucleotide as a primer and the like, or other methods well known to one skilled in the art. These methods are described, for instance, in "Molecular Cloning A Laboratory Manual, Third Edition", Cold Spring Harbor Laboratory Press (2001).

### Examples

The present invention will be explained in further detail with reference to examples shown below.

### Example 1. Screening for strains having L-Ile hydroxylase

### <1> Screening for 4-hydroxyisoleucine producing strains and analysis of broth

By using L-isoleucine as a substrate, screening was performed for microorganisms having a 4-hydroxyisoleucine producing ability. In water, 0.4% (w/v) of soluble starch, 0.4% of yeast extract, 1% of malt extract and 0.2% of L-isoleucine were dissolved, then the solution was adjusted to pH 7 to 7.5 to obtain a medium, and soil bacteria were inoculated in the medium. After shaking culture at 28°C for 2 days, 4-hydroxyisoleucine was analyzed by amino acid analysis of the centrifugation supernatant.

### Amino acid analysis conditions

4-Hydroxyisoleucine was detected by using the Waters AccQ-Tag^{™} method. Amino acids in 5 µl of a reaction mixture diluted to an appropriate concentration were derivatized in a conventional manner, and the production amount of 4-hydroxyisoleucine was measured by HPLC analysis. As a result, one of the bacterial strains (strain 2-e-2) was found to have an activity for producing a substance showing the same retention time as that of 4-hydroxyisoleucine. On the basis of the analysis of 16S rDNA, the strain 2-e-2 was identified as *Bacillus thuringiensis.* Therefore, other *Bacillus* bacteria were also screened, and a similar activity was also found in *Bacillus licheniformis* (strain AKU 223, strain IAM 11054), *Bacillus sphaericus* (strain AKU 227, strain NBRC 3526) and *Bacillus thuringiensis* (strain AKU 238, strain NBRC 3958).

### Identification of product

The substance produced from L-isoleucine as a substrate by the *Bacillus* strains obtained in the aforementioned screening was identified. First, the molecular weight of the product was analyzed by MS, and found to be 145, which was smaller than that of 4-hydroxyisoleucine by 2. Further, when the composition formula was estimated by precise mass measurement using a high resolution mass spectrometer (Q-TofMS), C₆H₁₁NO₃ was obtained and found to have hydrogen atoms in a number fewer by 2 than that of 4-hydroxyisoleucine. The above results suggested that the substance produced by the aforementioned *Bacillus* strains might be 2-amino-3-keto-4-methylpentanoic acid (AMKP). According to the experimental method described in the report about production of AMKP by *Bacillus* bacteria (Bioorganic Chemistry, Vol. 6, pp.263-271, 1977), AMKP was synthesized and purified. Further, the product was purified from the culture media of the aforementioned *Bacillus* bacteria according to the AMKP purification method of Bioorganic Chemistry, Vol. 6, pp.263-271, 1977, and NMR analysis was performed. As a result, both showed similar chemical shifts.

From the above, it was revealed that the *Bacillus* bacteria found in this experiment produced AMKP. The amount of AMKP produced by the strain 2-e-2 was about 1 to 2 mM. On the other hand, the estimated amount of AMKP produced by the *Bacillus* bacteria described in Bioorganic Chemistry, Vol. 6, pp.263-271, 1977 was described to be 0.04 mM, and thus a high AMKP production activity of the strain 2-e-2 was confirmed.

### <2> Establishment of method for separation and analysis of AMKP and HIL

Since the *Bacillus* bacteria such as the strain 2-e-2 were found to have an AMKP production activity, it was found that it was necessary to establish a method for separation and analysis of AMKP and 4-hydroxyisoleucine. As a result of various examinations, a method for separation and analysis of AMKP and 4-hydroxyisoleucine was established by modifying the Waters AccQ-Tag™ method. Specifically, the column was changed to XBridge C18 5 mm, 2.1 x 150 mm (Waters), Eluent B was changed to MeOH, and the flow rate of the eluent was changed to 0.3 ml/min. The gradient of the eluent is shown in the following table.

**Table 2: Eluent conditions in simultaneous analysis of HIL and AMKP**

| Time (min) | Flow (ml/min) | Accq Tag Eluent A | 100% MeOH | H₂O | Curve |
|---|---|---|---|---|---|
| | | %A | %B | %C | |
| - | 0.3 | 80 | 20 | 0 | - |
| 15 | 0.3 | 60 | 40 | 0 | 6 |
| 15.1 | 0.3 | 0 | 60 | 40 | 11 |
| 18.0 | 0.3 | 80 | 20 | 0 | 11 |

Under the above conditions, AMKP in the medium was eluted around 11.0 minutes, 4-hydroxyisoleucine was eluted around 11.9 minutes, and therefore these products could be separated.

### <3> Change in production amount of AMKP by addition of cofactors during culture

As a mechanism of the AMKP production activity, the possibility of uptake of molecular oxygen by hydroxylation was considered. Accordingly, the AMKP production activity was analyzed with addition of NAD(P)H, which is a cofactor of monooxygenases, or Fe²⁺, 2-oxoglutaric acid and ascorbic acid, which are cofactors of dioxygenases, during the culture of the strain 2-e-2.

By using the culture in the medium used in the previous screening as a control, AMKP production activities were compared for a 10 mM NADP and 10 mM NADPH addition group, Fe²⁺ addition group, and Fe²⁺, 2-oxoglutaric acid and ascorbic acid addition group. The culture temperature was 30°C, and the culture time was 22 hours. The AMKP concentrations in the culture supernatants were measured. The results are shown in Table 3.

**Table 3: Effect of addition of cofactors on AMKP production activity in culture of strain 2-e-2**

| | AMKP concentration (mM) |
|---|---|
| Control | 1.34 |
| NAD(P)H | 1.58 |
| Fe²⁺ | 2.53 |
| Fe²⁺, 2-oxoglutaric acid, ascorbic acid | 3.06 |

It was suggested that a dioxygenase might be involved in the production process of AMKP.

### <4> Changes in strain 2-e-2 broth over time

An AMKP production medium (0.4% (w/v) of soluble starch, 0.4% of yeast extract, 1% of malt extract, 0.2% of L-isoleucine, 0.5% of glucose, 1 mM ascorbic acid, 1 mM 2-oxoglutaric acid, 1 mM CaCl₂, 1 mM MgSO₄, pH 7 to 7.5) was put into a 3-L Sakaguchi flask, and the strain 2-e-2 was cultured at 23°C with shaking. At 0, 6, 8, 10, 12, 14, 16, 18 and 20 hours after the start of the culture, the culture broth was sampled, and 4-hydroxyisoleucine and AMKP in the culture broth were quantified by the method described in <2> mentioned above. Further, turbidity (OD₆₆₀) was also measured.

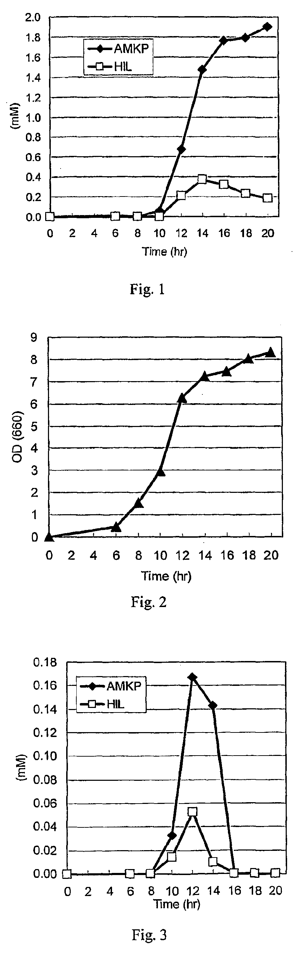

The concentrations of 4-hydroxyisoleucine (HIL) and AMKP are shown in Fig. 1. The turbidity of the culture broth is shown in Fig. 2. The sum of 4-hydroxyisoleucine and AMKP increased in the logarithmic growth phase and then reached the plateau. Further, after the level reached the plateau, 4-hydroxyisoleucine gradually decreased, and AMKP gradually increased.

At each of the aforementioned culture times, cells of the strain 2-e-2 were obtained from 200 µl of the culture broth, washed with physiological saline, and then suspended in 100 µl of a dioxygenase reaction mixture (10 mM Ile, 1 mM Fe²⁺, 10 mM 2-oxoglutaric acid, 10 mM ascorbic acid, 50 mM potassium phosphate buffer (pH 7.0)), the reaction was allowed at 30°C for 1 hour with shaking, and the production amounts of 4-hydroxyisoleucine and AMKP in the supernatant were measured. As a result, only when cells in the logarithmic growth phase were used, production of 4-hydroxyisoleucine and AMKP could be confirmed, although they were produced in trace amounts as shown in Fig. 3.

### <5> Production of HIL with strain 2-e-2 cell lysate

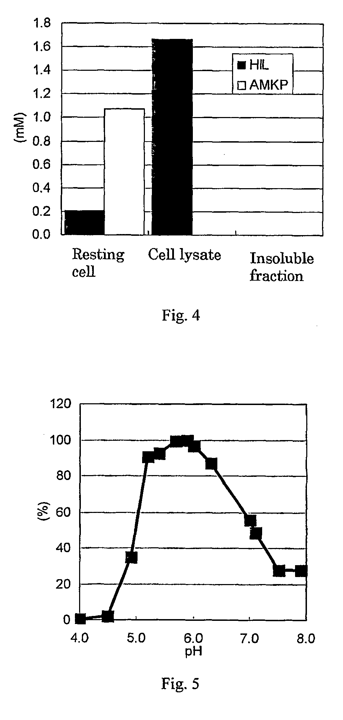

The cells were cultured in an AMKP production medium until OD₆₆₀ of 3.2 was achieved, the collected and washed strain 2-e-2 cells were disrupted in a mortar, and then suspended in a buffer for suspension (50 mM HEPES (pH 7.0), 10% of glycerol, Complete Mini (Roche)) to obtain a suspension (lysate) having a protein concentration of about 10 mg/ml, and centrifugation precipitates of the suspension was obtained. The centrifugation precipitates were finally suspended in physiological saline of the same volume as the cell lysate. These were each mixed with equal volume of a 2 x dioxygenase reaction mixture (10 mM Ile, 2 mM Fe²⁺, 10 mM 2-oxoglutaric acid, 10 mM ascorbic acid, 100 mM HEPES (pH 7.0)), and the reaction was allowed at 30°C for 1 hour. Similarly, resting cells used for the preparation of the cell lysate were washed, and suspended in physiological saline at a concentration 10 times that in the culture broth, the suspension was mixed with equal volume of the 2 x dioxygenase reaction mixture, and the reaction was allowed. The production amounts of AMKP and 4-hydroxyisoleucine in the samples are shown in Fig. 4.

By using the cell lysate, activity for 4-hydroxyisoleucine production using Ile as a substrate could be definitely confirmed.

### <6> Effects of cofactors for enzyme in strain 2-e-2 cell lysate

By using a cell lysate of the strain 2-e-2 cells in the logarithmic growth phase, effects of cofactors on the 4-hydroxyisoleucine production reaction by hydroxylation of Ile were examined. The reaction was allowed in a 50 mM HEPES (pH 7) reaction mixture containing 5 mM Ile and 5 mM of one of various cofactors in terms of final concentrations with a cell lysate of the strain 2-e-2 cells (lysate) in the logarithmic growth phase prepared by the method described in <5> mentioned above, and the production amount of 4-hydroxyisoleucine was measured. As shown in Table 4, Fe²⁺ (Fe) and 2-oxoglutaric acid (a-KG) were essential for the production of 4-hydroxyisoleucine, and the production amount of 4-hydroxyisoleucine was maximized by further adding ascorbic acid (Asc.). Therefore, it was strongly suggested that a dioxygenase might be involved in the production of 4-hydroxyisoleucine by hydroxylation of isoleucine.

**Table 4: Effects of various cofactors on 4-hydroxyisoleucine production activity with strain 2-e-2 cell lysate**

| Reaction mixture | HIL production (mM) |
|---|---|
| Lysate (-) | 0.00 |
| Lysate + Ile | 0.00 |
| Lysate + Ile + Fe | 0.00 |
| Lysate + Ile + Asc. | 0.00 |
| Lysate + Ile + a-KG | 0.00 |
| Lysate + Ile + Fe + Asc. | 0.00 |
| Lysate + Ile + Fe + a-KG | 0.39 |
| Lysate + Ile + Asc. + a-KG | 0.00 |
| Lysate + Ile + Fe + Asc. + a-KG | 0.85 |

### <7> Steric configuration of product obtained with strain 2-e-2 cell lysate

4-Hydroxyisoleucine has asymmetric carbons at 3 sites, and 8 types of diastereomers and 4 pairs of enantiomers exist. Specifically, the 4 pairs of enantiomers are (2S,3S,4S) and (2R,3R,4R) enantiomers (henceforth also referred to as HIL1), (2S,3S,4R) and (2R,3R,4S) enantiomers (henceforth also referred to as HIL2), (2S,3R,4R) and (2R,3S,4S) enantiomers (henceforth also referred to as HIL3), and (2S,3R,4S) and (2R,3S,4R) enantiomers (henceforth also referred to as HIL4). The naturally occurring HIL existing in Fenugreek and so forth is the (2S,3R,4S) enantiomer. Since (2S,3S)-isoleucine is used as a substrate in the present invention, 4-hydroxyisoleucine produced by the hydroxylation reaction is either the (2S,3R,4S) or (2S,3R,4R) enantiomer. Accordingly, it was decided to determine the steric configuration of 4-hydroxyisoleucine produced by the strain 2-e-2.

As the various enantiomer pairs, (2R,3R,4R): HIL1 and (2S,3R,4R): HIL3 were obtained according to Tetrahedron 47(32), 6469-6482, (1991), and (2R,3R,4S): HIL2 and (2S,3R,4S): HIL4 were obtained according to Eur. J. Org. Chem. 834-839, (2002). When HIL1 to HIL4 obtained by chemical synthesis were analyzed under the conditions of the simultaneous analysis of 4-hydroxyisoleucine and ANKP described in <2> mentioned above, the retention times were as shown in Table 5.

**Table 5: Retention times of diastereomers of 4-hydroxyisoleucine**

| | Retention time (min) |
|---|---|
| HIL1 | 7.25 |
| HIL2 | 14.28 |
| HIL3 | 11.10 |
| HIL4 | 11.98 |

When the 4-hydroxyisoleucine-containing sample prepared in <6> mentioned above was analyzed, the retention time was 11.99 minutes. When it was mixed with 4-hydroxyisoleucine of the standard of HIL4 and analyzed, the peaks completely matched. Therefore, it was shown that, when (2S,3S)-isoleucine was used as a substrate, 4-hydroxyisoleucine produced by this enzyme was HIL4, i.e., the naturally occurring 4-hydroxyisoleucine, among HIL3 and HIL4 possibly produced.

### <8> Optimum pH of enzyme in strain 2-e-2 cell lysate

By using a cell lysate prepared from strain 2-e-2 cells at OD₆₆₀ of 7 according to the method described in <5> mentioned above, pH dependency of the 4-hydroxyisoleucine production activity was evaluated. Composition of the dioxygenase reaction mixture consisted of 5 mM Ile, 5 mM Fe²⁺, 5 mM 2-oxoglutaric acid, 5 mM ascorbic acid and 100 mM GTA, and pH of the reaction mixture was measured after the reaction. The reaction temperature was 30°C. The produced 4-hydroxyisoleucine was analyzed by the method mentioned in <2>. Activities at various pH values are shown in Fig. 5 in terms of relative activity ratios calculated on the basis of the amount of HIL produced at pH providing the maximum production amount, which was taken as 100%. The activity was confirmed at pH 5 to 8.

### <9> Optimum temperature of enzyme in strain 2-e-2 cell lysate

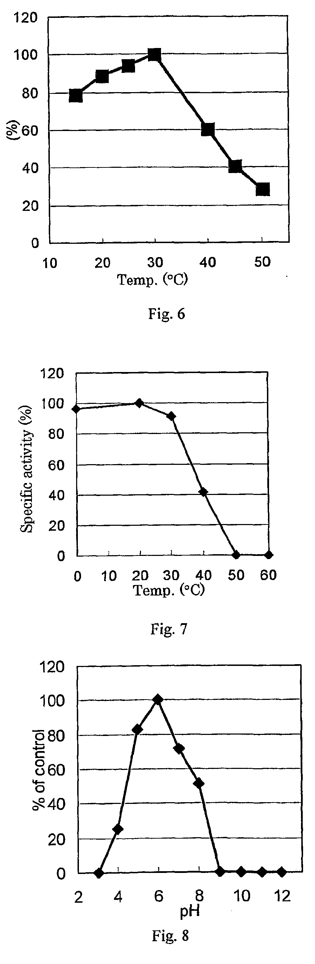

By using a cell lysate prepared from strain 2-e-2 cells at OD₆₆₀ of 7 according to the method described in <5> mentioned above, temperature dependency of the 4-hydroxyisoleucine production activity was evaluated. Composition of the dioxygenase reaction mixture consisted of 5 mM Ile, 5 mM Fe²⁺, 5 mM 2-oxoglutaric acid, 5 mM ascorbic acid and 100 mM GTA (pH 6), and the reaction temperature was 15 to 50°C. The produced 4-hydroxyisoleucine was analyzed by the method mentioned in <2>. Activities at various temperatures are shown in Fig. 6 in terms of relative activity ratios calculated on the basis of the amount of 4-hydroxyisoleucine produced at the temperature providing the maximum production amount, which was taken as 100%. The optimum temperature was lower than 45°C.

### <10> Temperature stability of enzyme in strain 2-e-2 cell lysate

By using a cell lysate prepared from strain 2-e-2 cells at OD₆₆₀ of 7 according to the method described in <5> mentioned above, temperature stability of the 4-hydroxyisoleucine production activity was evaluated. The cell lysate was incubated at 0 to 50°C for 1 hour, and then the Ile hydroxylation activity was measured. Composition of the substrate reaction mixture consisted of 5 mM Ile, 5 mM Fe²⁺, 5 mM 2-oxoglutaric acid, 5 mM ascorbic acid and 100 mM HEPES (pH 7), and the reaction temperature was 30°C. The produced 4-hydroxyisoleucine was analyzed by the method mentioned in Example 2. Temperature stabilities at various temperatures are shown in Fig. 7 in terms of relative activity ratios calculated on the basis of the amount of 4-hydroxyisoleucine produced at the temperature providing the maximum production amount, which was taken as 100%. The enzyme was inactivated at 50°C or higher.

### <11> Substrate reaction characteristics of enzyme in strain 2-e-2 cell lysate

By using a cell lysate prepared from strain 2-e-2 cells at OD₆₆₀ of 7 according to the method described in <5> mentioned above, reaction characteristics for various amino acids were evaluated. The cell lysate and a substrate solution were mixed, then the reaction was allowed at 30°C for 1 hour, and production of new substances was evaluated by TLC or amino acid analysis. Composition of the substrate reaction mixture consisted of 5 mM amino acid, 5 mM Fe²⁺, 5 mM 2-oxoglutaric acid, 5 mM ascorbic acid and 100 mM HEPES (pH 7). In addition to L-isoleucine, L-leucine, L-valine, L-glutamic acid and L-lysine were individually evaluated as amino acids. The produced 4-hydroxyisoleucine was analyzed by the method mentioned in Example 1. The results are shown in Table 6. Production of new substance could not be confirmed for amino acids other than L-isoleucine. Therefore, it was suggested that this enzyme was an isoleucine-specific dioxygenase.

**Table 6: Reactivity for various amino acids**

| | Product |
|---|---|
| L-Isoleucine | Produced (HIL) |
| L-Leucine | None |
| L-Valine | None |
| L-Glutamic acid | None |
| L-Lysine | None |

### <12> Effect of inhibitors on enzyme in strain 2-e-2 cell lysate

By using a cell lysate prepared from strain 2-e-2 cells at OD₆₆₀ of 7 according to the method described in <5> mentioned above, effects of inhibitors on the 4-hydroxyisoleucine production activity were examined. A cell lysate prepared from strain 2-e-2 cells at OD₆₆₀ of 7 according to the method described in <5> mentioned above was used. Composition of the dioxygenase reaction mixture consisted of 5 mM Ile, 5 mM Fe²⁺, 5 mM 2-oxoglutaric acid, 5 mM ascorbic acid and 100 mM HEPES (pH 6), the reaction temperature was 30°C, and the reaction time was 1 hour. The amount of 4-hydroxyisoleucine produced when 10 mM of each inhibitor (EDTA, Cu²⁺, Zn²⁺) was added to the reaction system was measured. 4-Hydroxyisoleucine was analyzed by the method described in <2> mentioned above. The isoleucine hydroxylation activity was lost by the inhibitors.

### Example 2. Isolation and purification of L-Ile hydroxylase

### (1) Preparation of cell-free extract

The strain 2-e-2 was cultured in 2 L as a total volume of AMKP production medium until OD₆₆₀ of 6.0 was achieved, and then the cells were washed with physiological saline. The cells were suspended in a cell membrane treatment solution (10 mg/ml of lysing enzyme (SIGMA), 5 mg/ml of cellulase "ONOZUKA" R-10 (Yakult), Yatalase (Takara Bio), 1 mg/ml of lysozyme (SIGMA), dissolved in 0.2 M NaH₂PO₄ and 0.6 M KCl (pH 5.5)), and then incubated at 30°C for 1 hour. The incubated cells were washed with physiological saline, and then suspended in Buffer A (50 mM HEPES (pH 7.0), 10% of glycerol, 2 mM DTT, 1 mM EDTA, Complete (Roche)), and the cells were disrupted by using an ultrasonic disruptor (Branson) with ice cooling. This treated suspension was centrifuged at 4°C and 18,500 x g for 60 minutes to obtain a supernatant. The subsequent isolation and purification procedures were all performed at 4°C or with ice cooling.

### (2) Anion exchange chromatography

The supernatant obtained in the previous step was filtered through a filter with a pore size of 0.45 µm, and applied to a DEAE column (16 mm x 100 mm, GE Healthcare Bio-Sciences) equilibrated beforehand with Buffer A. The column was washed with Buffer A, and elution was performed with a linear concentration gradient of sodium chloride in Buffer B (50 mM HEPES (pH 7.0), 10% of glycerol, 2 mM DTT, 1 mM EDTA, 0.5 M NaCl, Complete (Roche)).

### (3) Detection of active fraction

Each fraction was used for the reaction using an Ile hydroxylation activity reaction mixture (100 mM HEPES (pH 6.0), 5 mM L-Ile, 5 mM Fe²⁺, 5 mM 2-oxoglutaric acid, 5 mM ascorbic acid at final concentrations) at 30°C for 30 minutes. The enzyme was inactivated at 100°C, and then the amount of 4-hydroxyisoleucine was quantified by the aforementioned AMKP and HIL separation and measurement method. 4-Hydroxyisoleucine referred to in the subsequent analysis means a substance substantially consisting only of the isomer having the same retention time as that of naturally occurring 4-hydroxyisoleucine. The enzyme activity that produces 1 nmol of HIL per minute was defined as 1 U.

### (4) Cation exchange chromatography

The active fraction obtained in the previous step was substituted with Buffer C (50 mM MES (pH 5.2), 10% of glycerol, 2 mM DTT, 1 mM EDTA, Complete (Roche)) in a desalting column (GE Healthcare Bio-Sciences). This substituted fraction was applied to a MonoS column (10 mm x 100 mm, GE Healthcare Bio-Sciences) equilibrated beforehand with Buffer C. The column was washed with Buffer C, then elution was performed with a linear concentration gradient of sodium chloride in Buffer D (50 mM MES (pH 5.2), 10% of glycerol, 2 mM DTT, 1 mM EDTA, 0.5 M NaCl, Complete (Roche)), and the Ile hydroxylation activity of each fraction was measured.

### (5) Ammonium sulfate precipitation

2 M Ammonium sulfate was added to the soluble fraction obtained in the previous step and dissolved. The solution was sufficiently stirred, and then fractionated into a soluble fraction and a precipitate fraction by centrifugation, and the precipitate fraction was dissolved in Buffer A. When the Ile hydroxylation activity of each fraction was measured, the activity was detected in the precipitate fraction.

### (6) Size exclusion chromatography

The active fraction obtained in the previous step was applied to a Superdex 75 column (10 mm x 300 mm, GE Healthcare Bio-Sciences) equilibrated beforehand with Buffer A. Elution was performed with Buffer A, and the Ile hydroxylation activity of each fraction was measured.

### (7) Hydrophobic interaction chromatography

The active fraction obtained in the previous step was substituted with Buffer E (50 mM MES (pH 6.5), 10% of glycerol, 2 mM DTT, 1 mM EDTA, 1 M ammonium sulfate, Complete (Roche)). The buffer substituted sample was applied to a Resource PHE column (1 ml, GE Healthcare Bio-Sciences) equilibrated beforehand with Buffer E. The column was washed with Buffer E, and then a fraction containing the enzyme having the Ile hydroxylation activity was eluted with Buffer F (50 mM MES (pH 6.5), 10% of glycerol, 2 mM DTT, 1 mM EDTA, Complete (Roche)) by using a reverse linear concentration gradient of ammonium sulfate.

The outline of the isolation and purification of L-Ile hydroxylase are summarized in Table 7.

**Table 7: Summary of isolation and purification**

| Fraction | Total protein (mg) | Specific activity (U/mg) | Total activity (U) | Yield (%) |
|---|---|---|---|---|
| Cell-free extract | 678.173 | 3.8 | 2593.7 | 100.0 |
| DEAE | 73.324 | 17.8 | 1306.5 | 50.4 |
| monoS | 3.639 | 96.5 | 351.0 | 13.5 |
| Ammonium sulfate precipitation | 0.578 | 209.5 | 121.1 | 4.7 |
| GPC | 0.093 | 1222.8 | 113.4 | 4.4 |
| Resource PHE | 0.004 | 3694.9 | 13.2 | 0.5 |

### Example 3. Characterization of L-Ile hydroxalase

### <1> Analysis by electrophoresis

The purified sample obtained in Example 2 was analyzed by sodium dodecylsulfate-polyacrylamide gel electrophoresis (polyacrylamide gel: PAG Mini "Daiichi" 15/25 (13 wells) produced by Daiich Pure Chemicals Co., Ltd., molecular weight standards: Prestained SDS-PAGE Standards, Low Range, produced by Bio-Rad). As a result, it was found that the enzyme consisted of substantially uniform subunits having a molecular weight of about 31,000 ± 20,000.

### <2> Effects of addition of cofactors

Effects of cofactors on the 4-hydroxyisoleucine production reaction by hydroxylation of Ile were examined by using the purified enzyme. The L-Ile hydroxylase prepared by the method described in Example 2 was used for the reaction in a 100 mM HEPES (pH 6) reaction mixture containing 5 mM L-Ile and 5 mM of one of various cofactors at final concentrations, and the production amount of 4-hydroxyisoleucine was measured. As shown in Table 8, Fe²⁺ and 2-oxoglutaric acid were essential for the production of 4-hydroxyisoleucine, and the production amount of 4-hydroxyisoleucine was maximized by further adding ascorbic acid. Therefore, it was strongly suggested that a dioxygenase might be involved in the production of 4-hydroxyisoleucine by hydroxylation of L-isoleucine. This result was the same as the result of the examination using a cell lysate.

**Table 8: Effects of various cofactors on 4-hydroxyisoleucine production activity of purified enzyme**

| Extract | Substrate (L-Ile) | Cofactor | HIL (mM) |
|---|---|---|---|
| + | - | - | 0.00 |
| + | + | - | 0.00 |
| + | + | *α*-KG | 0.00 |
| + | + | Ascorbate | 0.00 |
| + | + | Fe²⁺ | 0.00 |
| + | + | α-KG + ascorbate | 0.00 |
| + | + | α-KG + Fe²⁺ | 0.02 |
| + | + | Ascorbate + Fe²⁺ | 0.00 |
| + | + | α-KG + ascorbate + Fe²⁺ | 0.26 |

### <3> Optimum pH

By using L-Ile hydroxylase prepared according to the method described in Example 2, pH dependency of the 4-hydroxyisoleucine production activity was evaluated. Composition of the enzymatic reaction solution consisted of 5 mM L-Ile, 5 mM Fe²⁺, 5 mM 2-oxoglutaric acid, 5 mM ascorbic acid and 200 mM GTA (pH 3 to 12). The reaction temperature was 30°C. Activities at various pH values are shown in Fig. 8 in terms of relative activity ratios calculated on the basis of the amount of 4-hydroxyisoleucine produced at pH providing the maximum production amount, which was taken as 100%. The activity was confirmed at pH 4 to 8, and high activity was confirmed at pH 5 to 8.

### <4> Optimum temperature

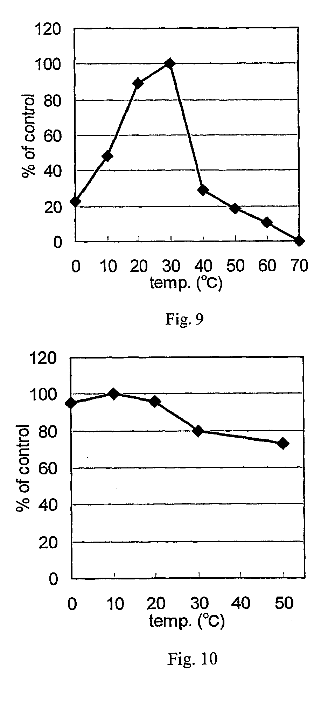

By using L-Ile hydroxylase prepared according to the method described in Example 2, optimum temperature of the 4-hydroxyisoleucine production activity was evaluated. Composition of the enzymatic reaction solution consisted of 5 mM Ile, 5 mM Fe²⁺, 5 mM 2-oxoglutaric acid, 5 mM ascorbic acid and 100 mM HEPES (pH 6). Activities at various temperatures are shown in Fig. 9 in terms of relative activity ratios calculated on the basis of the amount of 4-hydroxyisoleucine produced at the temperature providing the maximum production amount, which was taken as 100%. High activity was confirmed in the temperature range of 0 to 40°C.

### <5> Temperature stability

By using L-Ile hydroxylase prepared according to the method described in Example 2, temperature stability of the 4-hydroxyisoleucine production activity was evaluated. The enzyme solution at pH 7.0 was incubated at 0 to 70°C for 1 hour, and then the Ile hydroxylation activity was measured. Composition of the enzymatic reaction solution consisted of 5 mM Ile, 5 mM Fe²⁺, 5 mM 2-oxoglutaric acid, 5 mM ascorbic acid and 100 mM HEPES (pH 6), and the reaction temperature was 30°C. Temperature stabilities at various temperatures are shown in Fig. 10 in terms of relative activity ratios calculated on the basis of the amount of hydroxyisoleucine produced at the storage temperature providing the maximum production amount, which was taken as 100%. The enzyme was inactivated at 60°C or higher.

### <6> Substrate reaction characteristics

By using L-Ile hydroxylase prepared according to the method described in Example 2, reaction characteristics for various amino acids were evaluated. The enzyme solution and the reaction mixture were mixed, and then the reaction was allowed at 30°C for 1 hour, and production of new substances was evaluated by HPLC analysis. Composition of the enzymatic reaction solution consisted of 5 mM amino acid, 5 mM Fe²⁺, 5 mM 2-oxoglutaric acid, 5 mM ascorbic acid and 100 mM HEPES (pH 6). In addition to L-isoleucine, D-isoleucine, L-leucine, L-valine, L-glutamic acid and L-lysine were individually evaluated as amino acids. The produced 4-hydroxyisoleucine was analyzed by the method of Example 1. The results are shown in Table 9. Like the results of the examination using a cell lysate, production of new substance could not be confirmed for amino acids other than L-isoleucine. Therefore, it was suggested that the enzyme was L-isoleucine-specific dioxygenase, and had an activity for producing naturally occurring HIL, as similarly shown by the results of the examination using a cell lysate.

**Table 9: Reactivity for each amino acids**

| | Product |
|---|---|
| L-Isoleucine | Produced (HIL) |
| D-Isoleucine | None |
| L-Leucine | None |
| L-Valine | None |
| L-Glutamic acid | None |
| L-Lysine | None |

### <7> Effects of inhibitors

By using L-Ile hydroxylase prepared according to the method described in Example 2, effects of inhibitors on the 4-hydroxyisoleucine production activity were examined. Composition of the enzymatic reaction solution consisted of 5 mM Ile, 5 mM Fe²⁺, 5 mM 2-oxoglutaric acid, 5 mM ascorbic acid and 100 mM HEPES (pH 6), the reaction temperature was 30°C, and the reaction time was 1 hour. The amount of 4-hydroxyisoleucine produced when 10 mM of each inhibitor (EDTA, Cu²⁺, Zn²⁺) was added to the reaction system was measured. 4-Hydroxyisoleucine production activities observed with addition of the inhibitors are shown in Table 10 in terms of relative activities to the 4-hydroxyisoleucine production activity observed with no addition of inhibitor, which was taken as 100%. The isoleucine hydroxylation activity was lost by the inhibitors.

**Table 10: Effect of inhibitors**

| Inhibitor | Relative activity (%) |
|---|---|
| None | 100 |
| EDTA | 0 |
| Cu²⁺ | 0 |
| Zn²⁺ | 0 |

### <8> N-terminus amino acid sequence

L-Ile Hydroxylase prepared according to the method described in Example 2 was subjected to electrophoresis according to the method of Example 3, transferred to a PVDF membrane (sequi-Blot^{™} PVDF membrane, Bio-Rad), and used in PPSQ-10, which is a protein sequencer produced by Shimadzu Corporation. The following result was obtained as the N-terminal sequence of the enzyme obtained by the aforementioned method.

### Example 4. Purification of IDO from Bacillus thuringiensis (2-e-2) strain.

Screening of environmental microorganisms provided by inventors of present invention revealed unique microbe possessing α-ketoglutarate-dependent L-isoleucine dioxygenase activity. It was identified as *Bacillus thuringiensis* and stocked as *Bacillus thuringiensis* strain 2-e-2 (FERM BP-10688).
**1.1. Cultivation conditions.** The following cultivation medium were used in experiments:
   CM1 (Tryptone 10g/l; Yeast extract 10 g/l; pH 7.0 adjusted by NaOH);
   CM6 (Tryptone 10 g/l; Yeast extract 40 g/l; pH 7.0 adjusted by NaOH).
**1.2. Inoculum preparation.** *Bacillus thuringiensis* strain 2-e-2 was cultivated overnight at 30°C in CM1 medium supplemented with 90 mM KPi buffer (90 mM KH₂PO₄ (pH 7) adjusted by KOH). Then, glycerol was added to the grown biomass up to 20%. Obtained cells suspension was aliquoted in 1.9 ml vials and stored at -70°C until used.
**1.3. Fermentation conditions.** *Bacillus thuringiensis* strain 2-e-2 fermentation was carried out using Marubischi fermenters. The following cultivation parameters were used: starting culture volume - 600 ml; agitation - 800 rev/min; air -1:1; pH 7.0 was stabilized using 1H NaOH/HCl feeding. Frozen suspension (see previously item) from one vial (1.9 ml) was used to inoculate 600 ml of CM6 medium. Strain 2-e-2 was cultivated for about 7.5 hours. Then, cells were harvested by centrifugation and stored at -70 °C until used.
**1.4. IDO activity assay.** Cells from 25-100 ml of *Bacillus thuringiensis* strain 2-e-2 culture were harvested by centrifugation at 4°C and re-suspended in 2ml of buffer A (50 mM MOPS, 10% glycerol, 1 mM EDTA, 1 mM DTT, protease inhibitor, pH 7.2). Cells were disrupted using French-pressure cell (3X at 1000 Psi). Reaction mixture (50 µl) contained 50 mM HEPES pH 7.0; 5 mM Ile; 5 mM ascorbate; 10 mM FeSO₄; 5mM α-ketoglutarate and aliquot of protein preparation. Reaction was incubated at 34 °C for 40 min with shaking. Synthesized 4HIL was detected using TLC analysis. Thin-layer silica gel plate (10x15 cm) spotted with aliquot (1-2µl) of the reaction solution was developed with a developing solvent (2-propanol: acetone: ammonia: water =100:100: 25:16) and 4HIL was detected with the ninhydrin reagent. HPLC analysis was carried out as described in Example 4.
**1.5. Purification and identification of IDO from** *Bacillus thuringiensis* **strain2-e-2.**

All chromatographic procedures were carried out using ÄKTAbasic100 system (Amersham Pharmacia Biotech). Purification protocol includes the following steps. Frozen cells (62 g of weight biomass) were thawed and re-suspended in 150 ml buffer A [50 mM TRIZMA, 5% glycerol, 1 mM EDTA, 1 mM DTT, protease inhibitor, pH 7 adjusted by HCl].

*Step1*. Cells were disrupted by 2 passages through French pressure cell (max P = 1000 Psi) followed by centrifugation (14000 g, 4°C, 20 min) to remove cell debris. The obtained protein preparation was added to the 150 ml of the DEAE resin equilibrated in buffer A. Final suspension was incubated at 4°C for about 10 minutes with softly shaking. Then, resin with adsorbed protein was transferred to the column (26X30 cm) and two-step protein elution was carried out. At first, column was washed by 50 mM NaCl in buffer A. Then, IDO activity was eluted by 250 mM NaCl in buffer A.

*Step2.* IDO activity was concentrated by ammonium sulphate precipitation (at 1.9 M) and resuspendned in about 3 ml of buffer B[50 mM TRIZMA, 5% glycerol, 1 mM EDTA, 1 mM DTT, 100 mM NaCl, pH 7].

*Step3.* 1.5 ml of preparation obtained from *Step2* were applied to the Superdex 200 HR 10/30A column equilibrated with buffer B. Isocratic elution was performed at 0.5 ml/min flow rate. Active fractions were pooled.

*Step4.* The protein preparation obtained from *step 3* was applied to a 1.6 ml Sourse15Q column equilibrated with buffer A. The elution was carried out at flow rate 0.5 ml/min by liner gradient 0 - 0.5 M NaCl in buffer B (10 column volumes). Each 1 ml fraction was collected. Active fractions were pooled.

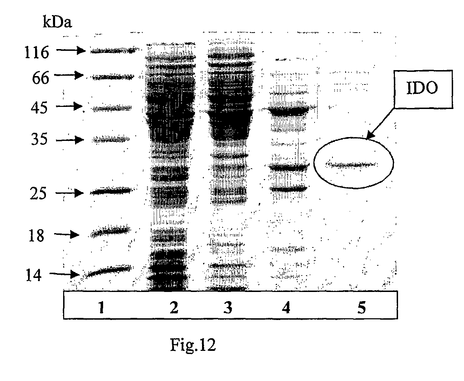

As a result, IDO was purified about 120-fold (see Table 11). SDS-PAGE of the final protein preparation revealed only one major band (about 70% of total protein, see Fig. 12).

| Table 11. Purification of the IDO from *Bacillus thuringiensis* strain 2-e-2. | | | | | |
|---|---|---|---|---|---|
| Step | Total protein (mg) | Total activity (nmoles/min) | Specific activity (nmoles/min/mg) | Yield (%) | Purification fold |
| Cell extract | 3072 | 19046 | 6 | 100 | 1 |
| DEAE (batch) | 1500 | 18750 | 12.5 | 98 | 2 |
| (NH₄)₂SO₄ | 109 | 12514 | 115 | 65 | 19 |
| Superdex 200 HR | 6 | 1356 | 226 | 7 | 38 |
| Source 15Q | 0.03 | 21 | 700 | 0.1 | 117 |

### 1.6 Identification of IDO.

Major protein was extracted from SDS-PAGE and analyzed by mass spectrometry analysis. Treatment of gels, trypsinolysis, protein extraction and mass analysis by time-of-flight matrix-assisted laser desorption-ionization (MALDI-TOF) were carried out according to protocols described by Govorun, V.M. et al, (The proteome comparative analysis of *Helicobacter pylori* clinical isolates. Biochemistry (Mosc), 68, 1, 42-49 (2003)). Protein was identified by the set of its photolytic peptide masses using Peptide Fingerprint option of Mascot software (Matrix Science, USA). The analyzed protein was identified by MS-analysis as a putative RBTH_06809 protein from Bacillus thuringiensis (serovar israelensis; ATCC 35646) strain (Fig. 13).

### Example 5. Cloning IDO genes from Bacillus thuringiensis strain 2-e-2 and Bacillus thuringiensis (serovar israelensis, ATCC 35646) strains.

The purified sample was electrophoresed in an SDS (15-25%) polyacrylamide gel. Protein transfer was performed during 1 h at 1 mA cm² at room temperature onto a polyvinylidenedifluoride membrane. After being stained with CBB, IDO band was cut out and subjected to automated Edman degradation performed with protein sequencer model PPSQ-10 (Shimadzu Co. Ltd., Kyoto,Japan). The first 20 N-terminal amino acids of the purified protein were determined.

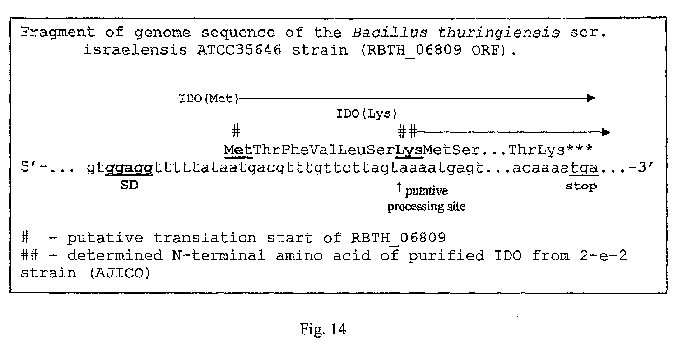

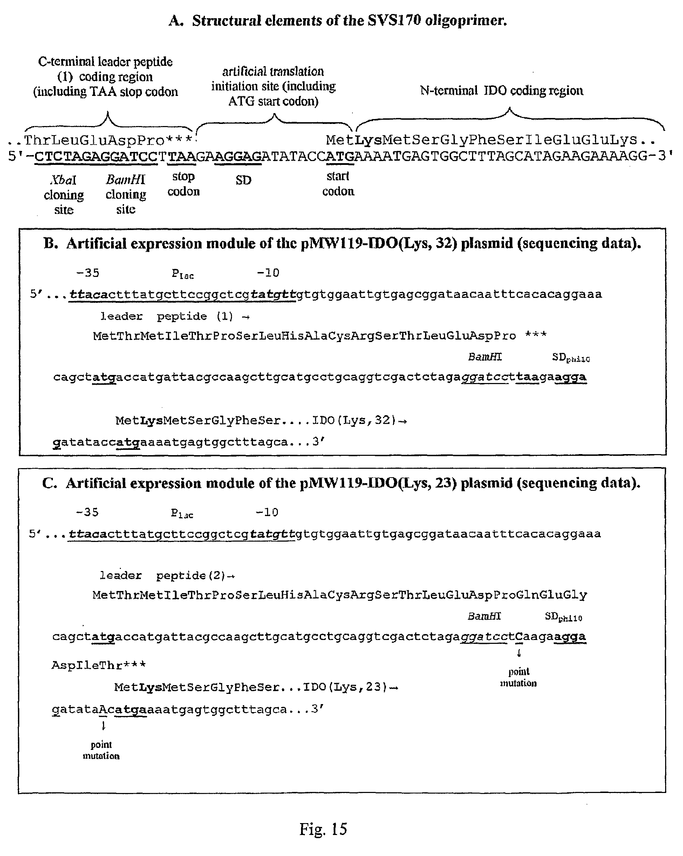

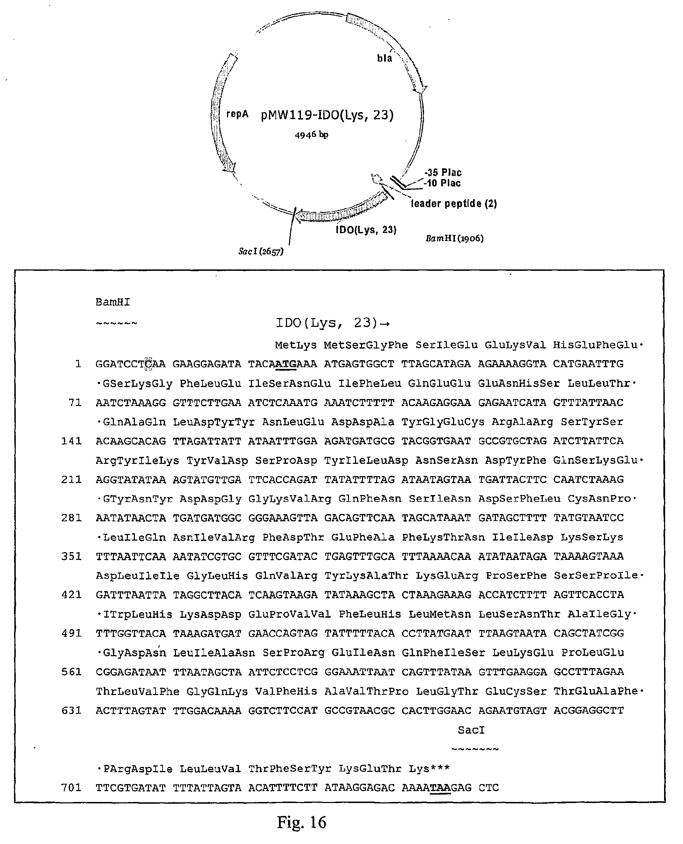

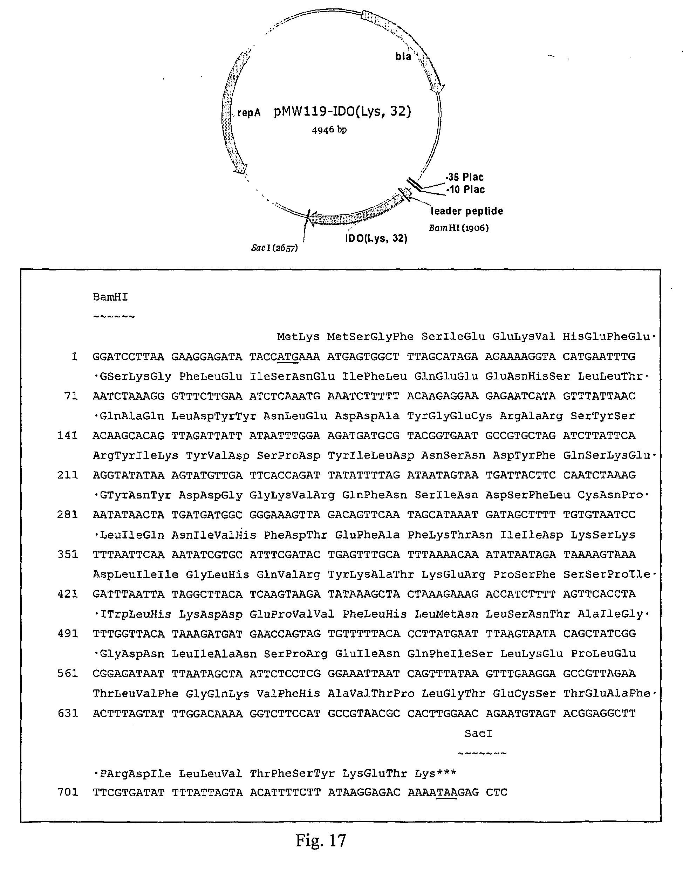

The N-terminal amino acid sequence of the 31-kDa polypeptide was determined to be KMSGFSIEEKVHEFESKGFL (SEQ ID NO: 5). After the BLAST search, this amino acids sequence showed high homology with hypothetical proteins of RBTH_06809 from *Bacillus thuringiensis* (serovar israelensis) ATCC 35646 and BC1061 from *Bacillus cereus* ATCC 14579 as shown in Table 1. Sequence analysis of the RBTH_06809 ORF and determination of N-terminal amino acid of purified protein indicate that there is only one potential IDO translation start because classic SD sequence is located at 8 bp upstream of ATG start codon (Fig. 14). However, Lys(7) is an N-terminal amino acid of purified protein. It was proposed that N-terminal processing (cleavage of N-terminal amino acids) is needed for IDO activity. Seemingly, such cleavage is the attribute of specific protease which co-expresses with IDO in *Bacillus sp.* but is lacking in *E*. *coli.* So, to produce mature IDO in *E*. *coli* special recombinant plasmids were constructed.
**2.1. Bacteria.** *Bacillus thuringiensis* (serovar israelensis, ATCC 35646) strain was obtained from Russian Collection of Industrial Microorganisms (VKPM), accession number B-197.
**2.2 Construction of the pMW119-IDO(Lys, 32) plasmid.** To construct pMW119-IDO(Lys, 32) the following procedures were carried out.
0.8 kb DNA fragment of the chromosome of the *Bacillus thuringiensis* (serovar israelensis, ATCC 35646) strain was amplified using oligonucleotides SVS 170 (SEQ ID No:3) and SVS 169 (SEQ ID No:4) as a primers (for detail see Fig. 15) and purified chromosomal DNA as a template. The following PCR protocols was used: initial cycle for 30 seconds at 94 °C; 4 cycles for 40 seconds at 94 °C; 30 seconds at 49 °C; 40 seconds at 72 °C; 35 cycles for 30 seconds at 94 °C; 30 seconds at 54 °C; 30 seconds at 72°C. Obtained PCR-fragment was digested with *BamH*I and *Sac*I endonucleases and then ligated into pMW119 vector previously treated with the same restrictases.
**2.3 Construction of the pMW119-IDO(Lys, 23) plasmid.** 0.8 kb DNA fragment of the chromosome of the *Bacillus thuringiensis* strain 2-e-2 was amplified using oligonucleotides SVS 170 (SEQ ID No:3) and SVS 169 (SEQ ID No:4) as a primers (for detail see Fig. 15) and purified chromosomal DNA of as a template. The following PCR protocols was used: initial cycle for 30 seconds at 94 °C; 4 cycles for 40 seconds at 94 °C; 30 seconds at 49 °C; 40 seconds at 72 °C; 35 cycles for 30 seconds at 94 °C; 30 seconds at 54 °C; 30 seconds at 72 °C. Obtained PCR-fragment was digested with *BamH*I and *Sac*I endonucleases and then ligated into pMW119 vector previously treated with the same restrictases.
Cells of *E. coli* strain TG1 were transformed by ligation mixtures. Resulting clones were selected on the X-gal/IPTG agar-plate (blue/white test). Then, IDO activity was tested in crude cell lysates of selected clones.
As a result, two clones TG1 [pMW119-(Lys, 32)] and TG1 [pMW119-(Lys, 23)] were selected. Corresponding plasmids were isolated and sequence analysis of cloned *BamH*I-*Sac*I fragments for each plasmid was carried out (see Fig. 16, Fig. 17).
Analyses of determined DNA sequences revealed discrepancy between cloned genes and known RBTH_06809 ORF (Fig. 18, Fig. 19). In addition, point mutation in regulatory region of IDO(Lys, 23) on the plasmid pMW119 was determined (Fig. 15 C) resulting in elimination TAA stop codon of leader peptide (1) and prolongation of its translation of up to TGA stop codon (leader peptide (2)) which is overlapped with ATG start codon (see Fig 15A, C). Additional mutation was detected in the "-2" position, where C was substituted by A (see Fig 15,C).
**2.4. IDO activity assay in crude cell lysate of TG1[pMW119-IDO(Lys, 23) and TG1[pMW119-IDO(Lys, 32) starns.** To investigate IDO activity in crude cell lysate of recombinant *E*. *coli* strains, the following procedures were carried out. Cells from 5 ml of culture were harvested by centrifugation at 4 °C, re-suspended in 0.5 ml of buffer A*(50 mM TRIZMA, 5% glycerol, 1 mM EDTA, 1 mM DTT, pH 7 adjusted by HCl) and disrupted by sonication at 4°C. Reaction mixture (50 µl) contained 50 mM HEPES pH 7.0; 5 mM Ile; 0.5mM α-ketoglutarate; 5 mM ascorbate; 5 mM FeSO₄ and aliquot of protein preparation. Reaction was incubated at 34°C for 1 hour with shaking. Synthesized 4HIL was detected using TLC or HPLC analysis as described in Example 4. Results are summarized in Table 12.

| Table 12. | |
|---|---|
| Strain | IDO activity (nmoles/mg*min) |
| TG1[pMW119] | ND^{a)} |
| TG1[pMW119-IDO(Lys, 23)] | 15 |
| TG1[pMW119-IDO(Lys, 32)] | 3 |
| a) ND - non detected ((≤0.03 nmoles/mg*min) | |

### Example 6. Biotransformation of L-isoleucine into 4HIL using IDO activity.

Cells of recombinant *E*. *coli* strains TG1[pMW119-IDO(Lys, 23)] and TG1[pMW119-IDO(Lys, 32)] were cultivated in LB medium supplemented ampicillin (100 mg/l), until optical density A₅₄₀ = 4-5 was reached (approximately 6 hours). After that cells from 2 ml culture broth were harvested by centrifugation and resuspended in 1ml of solution MI50 (100 mM KH₂PO₄ (pH 7 adjusted by NaOH), NH₄Cl 20 mM, MgSO₄ 2 mM, CaCl₂ 0.1 mM, ampicillin 150 µg/ml, Ile 50 mM, 0,5mM α-ketoglutarate, glycerol 1 %, yeast extract - 0.005 g/l).

Then, cells were cultivated for about 12 hours. After that, concentration of 4HIL was investigated by TLC (HPLC) analysis. Obtained data are summarized in the Table 13. As it can be seen from Table 12, TG1[pMW119-IDO(Lys, 23)] caused the accumulation of a higher amount of 4HIL as compared with TG1 [pMW119-IDO(Lys, 32)].

| Table 13. | | | | | |
|---|---|---|---|---|---|
| Strain | OD540 | | Ile supplied (mM) | 4HIL obtained (mM) | Yield^{b)} (%) |
| | 0 h | 12h | | | |
| TG1[pMW119] | 10 | 20 | 50 | ND^{a)} | - |
| TG1[pMW119-IDO(Lys, 23)] | 10 | 20 | 50 | 7 | 14 |
| TG1[pMW119-IDO(Lys, 32)] | 10 | 20 | 50 | 5 | 10 |
| a) ND - non detected (≤ 0.02 mM) | | | | | |
| b) Yield was calculated as (4HIL obtained/Ile supplied)* 100 | | | | | |

### Example 7. HPLC measurement of accumulated 4-hydroxy-L-isoleucine

HPLC analysis: High pressure chromatograph (Waters, USA) with spectrofluorometer 1100 series (Agilent, USA) was used. The chosen detection wave range: excitation wavelength at 250 nm, range of emission wavelengths were 320-560 nm. The separation by accq-tag method was performed in a column Nova-Pak^{™} C18 150 x 3.9 mm, 4µm (Waters, USA) at +400 °C. Injection volume of the sample was 5µl. The formation of amino acid derivatives and their separation was performed according to Waters manufacturer's recommendation (Liu, H. et al, J. Chromatogr. A, 828, 383-395 (1998); Waters accq-tag chemistry package. Instruction manual. Millipore Corporation, pp.1-9 (1993)). To obtain amino acid derivatives with 6-aminoquinolil-N-hydroxysuccinymidyl carbamate, the kit Accq-Fluor^{™} (Waters, USA) was used. The analysis by accq-tag method was performed using concentrated Accq-tag Eluent A (Waters, USA). All solutions were prepared using Milli-Q water, standard solutions were stored at + 4 °C.

### Example 8. Cloning of ido genes from Bacillus cereus ATCC 14597, B. thuringiensis AKU238, and B. weihenstephanensis KBAB4.

### (1) Preparation of chromosomal DNA

*Bacillus cereus* ATCC 14579, *B. thurigiensis* AKU238, and *B. weihenstephanensis* KBAB4 were each cultivated overnight at 28°C in 5 ml of LB medium (pre-culture). By using 1.5 ml of the culture broth as a seed, a main culture was carried out in 50 ml of LB medium. After cultivation up to a logarithmic growth phase, cells were harvested from 50 ml of the culture broth by centrifugation (12000x g, 4°C, 15 min). From these cells, chromosomal DNA was prepared according to an ordinary method.

### (2) Obtainment of ido genes by PCR

Based on published genomic sequence information about *B. cereus* ATCC 14579 (GenBank accession No. AE016877), the following primers were synthesized:
CATATGGAGGTTTTTATAATGACGTTTGTT (SEQ ID NO: 10)
CTCGAGTTTTGTCTCCTTATAAGAAAATGT (SEQ ID NO: 11)

By using the prepared primers and chromosomal DNA of *B. cereus* ATCC 14578 as a template, amplification by PCR was carried out with PrimeSTAR (TaKaRa) under the following condition: 30 cycles for 10 seconds at 98°C, 15 seconds at 52°C and 1 minute at 72°C.

The obtained PCR product was subjected to agarose gel electrophoresis and amplification of a fragment of about 750 bp was observed. The DNA fragment was collected, digested with *Nde*I and *Xho*I endonucleases, and cloned into an expression vector pET21b (Novagene) which has been digested with the same endonucleases. Then the nucleotide sequence was determined and its encoding amino acid sequence was deduced (SEQ ID NOS: 12 and 13). As a result, it was confirmed that a homologous *ido* gene was obtained based on the homology of the obtained DNA fragment. The homology at nucleotide sequence level was 98% relative to the *ido* gene from *B. thuringiensis israelensis*) ATCC 35646 and 98% relative to the *ido* gene from *B. tuhringiensis* 2-e-2.

As for *B. thuringiensis* AKU238, by the primers (SEQ ID NOS: 14 and 15) which were used for cloning of the *ido* gene from *B. thuringiensis* 2-e-2, an *ido* gene was cloned by PCR and sequenced in a similar manner to the above (SEQ ID NOS: 16 and 17). The homology at nucleotide sequence level was 98% relative to the *ido* gene from *B. thuringiensis israelensis* ATCC 35646 and 98% relative to the *ido* gene from *B. tuhringiensis* 2-e-2.
CATATGAAAATGAGTGGCTTTAGCATAGAA (SEQ ID NO: 14)
CTCGAGTTTTGTCTCCTTATAAGAAAATGT (SEQ ID NO: 15)

As for *B. weihenstephanensis* KBAB4, by the primers based on published genomic sequence information about *B. weihenstephanensis* KBAB4 (GenBank accession No. NZ_AAOY01000001), the following primers were synthesized: (SEQ ID NOS:18 and 19), an *ido* gene was cloned by PCR and sequenced in a similar manner to the above (SEQ ID NOS: 20 and 21). The homology at nucleotide sequence level was 78% relative to the *ido* gene from *B. thuringiensis israelensis* ATCC 35646 and 78% relative to the *ido* gene from *B*. *tuhringiensis* 2-e-2.
CATATGCTAACAACAGTTTCTAATAAGACA (SEQ ID NO: 18)
CTCGAGTTTTGGCTCCTTATAAGAAAACGT (SEQ ID NO: 19)

### Example 9. Expression of ido genes in E. coli and production of HIL from Ile

### (1) Expression of ido genes in E. coli

The plasmid expressing the *ido* gene from *B. cereus* ATCC 14579, *B. thuringiensis* AKU238, or *B. weihenstephanensis* KBAB4, which was constructed in Example 8, was introduced into *E*. *coli* Rosetta2 (DE3), and the resulting transformants were cultivated with shaking in LB medium supplemented with 50 µg/ml ampicilin (preculture). By seeding the preculture broth in 50 ml LB medium at 1%, the main culture was carried out at 37°C. At 2 hours after the start of the cultivation, IPTG was added at a final concentration of 1 mM, and the cultivation was carried out for further 3 hours. After completion of the cultivation, cells were harvested, washed, suspended in 1 ml of 20 mM Tris-HCl (pH 7.6) and disrupted with a sonicator (INSONATOR 201M, KUBOTA). The lysate was centrifuged at 15000 rpm for 10 minutes to obtain a supernatant which was used as a crude enzyme solution.

### (2) Production of HIL from Ile by using crude enzyme solution

By using the crude enzyme solution prepared in (1), the activity of converting Ile to HIL was measured. The reaction mixture had a composition as described below. The reaction mixture was reacted with shaking (300 rpm) at 28°C for 3 hours, and then the produced HIL was determined by HPLC. The determination results were shown in Table 14.

| Reaction mixture | |
|---|---|
| Cell lysate (Ultrasonic disruption) | 500 µl |
| 2% Ile | 100 µl |
| 1 M α-KG | 5 µl |
| 1 M Ascorbate | 5 µl |
| 0.1 M FeCl₂ | 10 µl |
| 1 M KPB (pH 6) | 100 µl |
| Total | 1 ml |

**Table 14 Production amount of HIL from Ile**

| | HIL (µM) |
|---|---|
| *B.thuringiensis* israelensis ATCC35646 | 2150 |
| *B.thuringiensis* 2e2 | 2321 |
| *B.thuringiensis* AKU238 | 2016 |
| *B.cereus* ATCC 14579 | 90 |
| *B.weihenstephanensis* KBAB4 | 3116 |
| control (pET21b) | 11 |

As results, when strains expressing expressing the *ido* gene from *B. cereus* ATCC 14579 and the plasmid expressing the *ido* gene from *B. thuringiensis* AKU238, or *B*. *weihenstephanensis* KBAB4 were used, the production of HIL were clearly observed. Thus, it was confirmed that these genes were usable for HIL production.

### Explanation of Sequences

1: Nucleotide sequence of IDO gene from B. thuringiensis strain 2-e-2
2: Amino acid sequence of IDO from B. thuringiensis strain 2-e-2
3: Primer svs 170; for amplification of IDO gene
4: Primer svs 169; for amplification of IDO gene
5: N-Terminal sequence of IDO from B. thuringiensis strain 2-e-2
6: IDO conserved sequence among Bacillus genus
7: Nucleotide sequence of IDO gene from B. thuringiensis strain ATCC 35646
8: Amino acid sequence of IDO from B. thuringiensis strain ATCC 35646
9: 16S rDNA nucleotide sequence of B. thuringiensis strain 2-e-2
10: Primer for amplification of IDO gene from B. cereus ATCC 14579
11: Primer for amplification of IDO gene from B. cereus ATCC 14579
12: Nucleotide sequence of IDO gene from B. cereus ATCC 14579
13: Amino acid sequence of IDO from B. cereus ATCC 14579
14: Primer for amplification of IDO gene from B. thuringiensis AKU238
15: Primer for amplification of IDO gene from B. thuringiensis AKU238
16: Nucleotide sequence of IDO gene from B. thuringiensis AKU23 8
17: Amino acid sequence of IDO from B. thuringiensis AKU238
18: Primer for amplification of IDO gene from B. weihenstephanensis KBAB4
19: Primer for amplification of IDO gene from B. weihenstephanensis KBAB4
20: Nucleotide sequence of IDO gene from B. weihenstephanensis KBAB4
21: Amino acid sequence of IDO from B. weihenstephanensis KBAB4

### INDUSTRIAL APPLICABILITY

According to the present invention, there is provided a method for producing 4-hydroxyisoleucine by using an enzyme derived from a microorganism and catalyzes a reaction producing 4-hydroxyisoleucine by direct hydroxylation of isoleucine. The present invention is extremely useful in the fields of pharmaceuticals and food.

The L-isoleucine dioxygenase used in the present invention catalyzes a hydroxylation reaction of L-isoleucine, and may be preferably used to synthesize (2S,3R,4S)-4-hydroxy-L-isoleucine, which is useful as a component of pharmaceutical compositions with insulinotropic activity.

## Claims

1. A method for producing 4-hydroxyisoleucine or a salt thereof, which comprises the step of producing 4-hydroxyisoleucine by subjecting isoleucine or a salt thereof to a hydroxylation reaction in the presence of a hydroxylase isolable from a microorganism and producing 4-hydroxyisoleucine from isoleucine,
wherein said hydroxylase requires Fe²⁺ and 2-oxoglutaric acid as cofactors.

2. The production method according to claim 1, wherein the microorganism is a microorganism belonging to the genus *Bacillus.*

3. The production method according to claim 2, wherein the microorganism belonging to the genus Bacillus is a microorganism selected from *Bacillus thuringiensis, Bacillus licheniformis, Bacillus sphaericus, Bacillus cereus,* and *Bacillus weihenstephanensis.*

4. The production method according to any one of claims 1 to 3, wherein the hydroxylation reaction is performed in the presence of a cell lysate prepared from microbial cells in a logarithmic growth phase.

5. The production method according to any one of claims 1 to 4, wherein the hydroxylase has the following properties:
(a) To require oxygen and ascorbic acid as cofactors,
(b) To have an optimum reaction pH of 5 to 8,
(c) To have an optimum reaction temperature of 45°C or lower,
(d) To be inactivated at 50°C or higher,
(e) To be inhibited by EDTA, Cu²⁺ and Zn²⁺.

6. A method for manufacturing (2S,3R,4S)-4-hydroxy-L-isoleucine or a salt thereof, comprising the steps of: reacting L-isoleucine in an aqueous solvent in the presence of at least one L-isoleucine dioxygenase or in the presence of a bacterium containing an L-isoleucine dioxygenase,
wherein the L-isoleucine dioxygenase is selected from the group consisting of:
(f) a protein comprising the amino acid sequence of SEQ ID No: 2, 8, 13, 17, or 21,
(g) a protein having an amino acid sequence that contains a substitution, deletion, insertion, addition or inversion of one to 13 amino acid residues in the amino acid sequence of SEQ ID No: 2, 8, 13, 17, or 21 and having L-isoleucine dioxygenase activity, and
(h) a protein that has at least 95% identity to the amino acid sequence of SEQ ID No: 2, 8, 13, 17, 21 and having L-isoleucine dioxygenase activity; isolating produced (2S,3R,4S)-4-hydroxy-L-isoleucine.

7. The method according to claim 6, wherein the bacterium is modified to enhance the activity of the L-isoleucine dioxygenase.

## Patentansprüche

1. Verfahren zum Herstellen von 4-Hydroxyisoleucin oder eines Salzes davon, das die Stufe umfasst, in der 4-Hydroxyisoleucin hergestellt wird, indem Isoleucin oder ein Salz davon einer Hydroxylierungsreaktion in Anwesenheit einer aus einem Mikroorganismus isolierbaren Hydroxylase unterworfen wird und 4-Hydroxyisoleucin aus Isoleucin hergestellt wird,
wobei die Hydroxylase Fe²⁺ und 2-Oxoglutarsäure als Cofaktoren erfordert.

2. Herstellungsverfahren nach Anspruch 1, wobei der Mikroorganismus ein Mikroorganismus der Gattung Bacillus ist.

3. Herstellungsverfahren nach Anspruch 2, wobei der Mikroorganismus der Gattung Bacillus ein Mikroorganismus ist, der unter Bacillus thuringiensis, Bacillus licheniformis, Bacillus sphaericus, Bacillus cereus und Bacillus weihenstephanensis ausgewählt ist.

4. Herstellungsverfahren nach einem der Ansprüche 1 bis 3, wobei die Hydroxylierungsreaktion in Anwesenheit eines aus einer mikrobiellen Zelle in einer logarithmischen Wachstumsphase hergestellten Zell-Lysats durchgeführt wird.

5. Herstellungsverfahren nach einem der Ansprüche 1 bis 4, wobei die Hydroxylase die folgenden Eigenschaften hat:
(a) sie benötigt Sauerstoff und Ascorbinsäure Cofaktoren,
(b) sie hat einen optimalen Reaktions-pH-Wert von 5 bis 8,
(c) sie hat eine optimale Reaktionstemperatur von 45°C oder niedriger,
(d) sie wird bei 50°C oder höher inaktiviert,
(e) sie wird durch EDTA, Cu²⁺ und Zn²⁺ inhibiert.

6. Verfahren zum Herstellen von (2S,3R,4S)-4-Hydroxy-L-isoleucin oder eines Salzes davon, welches die folgenden Stufen umfasst:
das Umsetzen von L-Isoleucin in einem wässerigen Lösungsmittel in Anwesenheit mindestens einer L-Isoleucindioxygenase oder in Anwesenheit eines eine L-Isoleucindioxygenase enthaltenden Bakteriums, wobei die L-Isoleucindioxygenase aus der Gruppe ausgewählt ist, die besteht aus
(f) einem Protein, das die Aminosäuresequenz der SEQ ID No: 2, 8, 13, 17 oder 21 aufweist,
(g) einem Protein mit einer Aminosäuresequenz, die eine Substitution, Deletion, Insertion, Addition oder Inversion von einem bis 13 Aminosäureresten in der Aminosäuresequenz der SEQ ID NO: 2, 8, 13, 17 oder 21 enthält und L-Isoleucindioxygenaseaktivität aufweist, und
(h) einem Protein, das mindestens 95% Identität zu der Aminosäuresequenz der SEQ ID No: 2, 8, 13, 17, 21 aufweist und L-Isoleucindioxygenaseaktivität hat;
und
das Isolieren des hergestellten (2S,3R,4S)-4-Hydroxy-L-isoleucins.

7. Verfahren nach Anspruch 6, wobei das Bakterium so modifiziert ist, dass die Aktivität der L-Isoleucindioxygenase erhöht ist.

## Revendications

1. Procédé de production de 4-hydroxyisoleucine ou d'un sel de celle-ci, qui comprend l'étape qui consiste à produire de la 4-hydroxyisoleucine en soumettant de l'isoleucine ou un sel de celle-ci à une réaction d'hydroxylation en présence d'une hydroxylase pouvant être isolée à partir d'un microorganisme, et à produire de la 4-hydroxyisoleucine à partir d'isoleucine,
où ladite hydroxylase nécessite du Fe²⁺ et de l'acide 2-oxoglutarique en tant que cofacteurs.

2. Procédé de production selon la revendication 1, dans lequel le microorganisme est un microorganisme appartenant au genre *Bacillus.*

3. Procédé de production selon la revendication 2, dans lequel le microorganisme appartenant au genre *Bacillus* est un microorganisme sélectionné parmi *Bacillus thuringiensis, Bacillus licheniformis, Bacillus sphaericus, Bacillus cereus,* et *Bacillus weihenstephanensis*.

4. Procédé de production selon l'une quelconque des revendications 1 à 3, dans lequel la réaction d'hydroxylation est réalisée en présence d'un lysat cellulaire préparé à partir de cellules microbiennes en phase de croissance logarithmique.

5. Procédé de production selon l'une quelconque des revendications 1 à 4, dans lequel l'hydroxylase possède les propriétés suivantes :
(a) elle doit nécessiter de l'oxygène et de l'acide ascorbique en tant que cofacteurs,
(b) elle doit posséder un pH de réaction optimal de 5 à 8,
(c) elle doit posséder une température de réaction optimale de 45 °C ou moins,
(d) elle doit être inactivée à 50 °C ou plus,
(e) elle doit être inhibée par l'EDTA, le Cu²⁺ et le Zn²⁺.

6. Procédé de fabrication de (2S, 3R, 4S)-4-hydroxy-L-isoleucine ou d'un sel de celle-ci, comprenant les étapes qui consistent à : faire réagir de la L-isoleucine dans un solvant aqueux en présence d'au moins une L-isoleucine dioxygénase ou en présence d'une bactérie contenant une L-isoleucine dioxygénase,
où la L-isoleucine dioxygénase est sélectionnée dans le groupe consistant en :
(f) une protéine comprenant la séquence d'acides aminés de SEQ ID NO: 2, 8, 13, 17, ou 21,
(g) une protéine ayant une séquence d'acides aminés qui contient une substitution, une délétion, une insertion, une addition ou une inversion de un à 13 résidus d'acides aminés dans la séquence d'acides aminés de SEQ ID NO:2, 8, 13, 17, ou 21 et ayant une activité L-isoleucine dioxygénase, et
(h) une protéine qui présente au moins 95 % d'identité avec la séquence d'acides aminés de SEQ ID NO: 2, 8, 13, 17, 21 et ayant une activité L-isoleucine dioxygénase ; isoler la (2S, 3R, 4S)-4-hydroxy-L-isoleucine produite.

7. Procédé selon la revendication 6, dans lequel la bactérie est modifiée pour améliorer l'activité de la L-isoleucine dioxygénase.
